# EUROPEAN PATENT APPLICATION

(11) **EP 4 190 347 A1**
(43) Date of publication of application: **07.06.2023**
(21) Application number: 21306683.0
(22) Date of filing: 01.12.2021
(51) Int. Cl.: A61K 38/39, A61K 38/10, A61P 9/00, A61P 11/00, A61P 25/28

(54) **SCO-SPONDIN-DERIVED POLYPEPTIDES FOR TREATING BIOLOGICAL BARRIERS DYSFUNCTION**

(71) Applicant: Axoltis Pharma, 63100 Clermont-Ferrand (FR)
(72) Inventor: LEMARCHANT, Sighild, Brunhilde, Adeline, 69008 LYON (FR); GODFRIN, Yann, 69300 CALUIRE (FR)
(74) Representative: Lavoix

(57) **Abstract**

The invention concerns polypeptides derived from SCO-spondin for treating a biological barrier dysfunction. More particularly the invention relates to said polypeptides for treating a biological barrier dysfunction in diseases or conditions comprising vascular diseases, infections, immune system hyper-responsiveness, inflammatory diseases or autoimmune diseases, chronic kidney disease, intestinal diseases, macular degeneration, diabetic macular edema, epilepsy, neuropathic pain, CNS diseases and disorders, and aging. The invention also relates to said polypeptides for treating a biological barrier dysfunction in a situation where a disease or condition is absent or not yet diagnosed.

## Description

The present invention concerns polypeptides derived from SCO-spondin for treating a biological barrier dysfunction. More particularly the invention relates to said polypeptides for treating a biological barrier dysfunction in diseases or conditions comprising vascular diseases, infections, immune system hyper-responsiveness, inflammatory diseases or autoimmune diseases, chronic kidney disease, intestinal diseases, macular degeneration, diabetic macular edema, epilepsy, neuropathic pain, CNS diseases and disorders, and aging. The invention also relates to said polypeptides for treating a biological barrier dysfunction in a situation where a disease or condition is absent or not yet diagnosed.

### Background of invention

Biological barriers protect organs and tissues from physical, chemical and biological damage and maintain homeostasis. In addition, biological barriers represent interfaces between organs and the "outside" may it be air for mucosal barriers or body fluids for vascular and brain barriers. Their main components are either endothelial or epithelial cell layers. The function of these cell layers is highly regulated by their microenvironment including neighboring cell types, the extracellular matrix or physical stimuli.

It is known that the functionality of biological barriers is altered in many diseases. And increasingly it is becoming evident that dysfunction of barriers is not only a symptom but in some instances is also causally related to disease progression. Biological barriers dysfunction can (1) be causally associated to the onset of a disease, (2) occur in parallel to additional physio-pathological mechanisms, (3) develop as a consequence of upstream events thereby generating additional complications (4) occur prior to the appearance of symptoms or to the diagnosis of a disease. Thus, biological barriers are attractive therapeutic targets and treating biological barriers dysfunction could be a therapeutic strategy applicable to a wide range of disorders.

The main physiological role of biological barriers is to maintain a selective permeability between organs and tissues and their "outside". Consequently, the main pathophysiological mechanism involving biological barriers is a loss of selective permeability allowing unwanted diffusion of pathogens, toxins, cells, inflammatory mediators, plasmatic proteins or drugs into tissues or organs.

Treating biological barriers dysfunction may thus consist in improving or restoring the selective permeability of said barriers. It may also be described as restoring barriers integrity or preventing barriers leakage.

Methods that could treat biological barriers dysfunction would be highly beneficial to patients with conditions comprising vascular diseases, infections, immune system hyper-responsiveness, inflammatory diseases or autoimmune diseases, chronic kidney disease, intestinal diseases, macular degeneration, diabetic macular edema, epilepsy, neuropathic pain, CNS diseases and disorders, and aging.

SCO-spondin-derived peptides have been described for their neuroprotective and neuroregenerative properties. The use of SCO-spondin-derived peptides for the treatment of spinal cord injury and tauopathies has been investigated in animal models. However, to date, no role has been suggested for their ability to treat biological barriers dysfunction.

Claudin-5 is enriched in barriers containing endothelial cells of cerebral (Greene et al., 2019; Hashimoto et al., 2021), retinal (Arima et al., 2020; Hudson et al., 2019; van der Wijk et al., 2019) and lung origins (Huang et al., 2016 ; Chen et al., 2013).

### Summary of the invention

The inventors surprisingly identified for the first time the ability of SCO-spondin-derived peptides at treating or preventing certain body dysfunctions in human and non-human animals. In a first aspect, the body dysfunction is or comprises a biological barrier dysfunction. In another aspect, the body dysfunction is or comprises altered tight junctions between adjacent cells forming the endothelial or epithelial layers of a biological barrier. In another aspect, the body dysfunction is or comprises transendothelial and/or transepithelial electrical resistance decrease. In another aspect, the body dysfunction is or comprises deregulation of a tight junction protein, especially of Claudin-5 expression or distribution. In another aspect, the body dysfunction is or comprises the trans-compartment (i.e., 2 physiological compartments separated by a biological barrier) permeability deregulation of specific compounds or molecules.

The permeability of biological barriers involves transcellular and paracellular permeability. Transcellular permeability is mediated by membrane receptors and vesicles whereas paracellular permeability is dependent upon adhesion mechanisms between adjacent cells forming the endothelial or epithelial layers of the barriers. Among these adhesion mechanisms, tight junctions are key contributors to restrict paracellular permeability in desired locations.

Tight junctions are cell adhesions consisting of multiple transmembrane proteins that directly interact via their extracellular components, linking the membranes of two adjacent cells membranes together. Several classes of proteins are involved in the tight junctional complex including members of the Claudins family which define the selectivity of paracellular permeability.

"Dysfunction of a barrier" means that the permeability of the barrier is modified with respect to normal (i.e. the barrier in a healthy subject), whose modification may be detrimental to good health, as this modification may lead to a change of permeability and crossing of compounds from the blood compartment to the parenchymal or fluid (e.g. cerebrospinal fluid) compartments, including blood elements such as inflammatory cytokines, fibrinogen and others (such as organic molecules, peptides, polypeptides, proteins, antibodies, viruses ...) with respect to a normal barrier. This dysfunction may in particular result from a disturbance of the tight junctions between endothelial cells and/or epithelial cells involved in the barrier, said disturbance leading to a permeability change.

In accordance with the invention, the biological barrier dysfunction may be treated in a situation where a disease or condition is absent or not yet diagnosed. The peptide administration may have a protective or preventive effect on the occurrence of a disease or disorder, or, in a situation where a disease or condition in the subject is known or suspected.

The present invention restores normal physiological barrier permeability or protects normal physiological barrier permeability or function. Permeability is recovered when the subject recovers normal permeability or when the subject recovers a permeability as close as possible to the permeability in the healthy subject. Permeability is protected (means the biological barrier is protected) when the peptide allows to keep permeability unchanged or to maintain permeability as close as possible to normal at the onset or during evolution of a disease that can deteriorate barrier permeability. In an aspect, the peptides used in the invention cause the endothelial cells of a barrier to get closer, the epithelial cells of a barrier to get closer, or the endothelial and epithelial cells of a barrier to get closer. In this aspect the tight junctions are said to be restored or improved. In another aspect, the peptides used in the invention induce an increase of Claudin-5 level at the surface of the barrier endothelial and/or epithelial cells, especially they cause Claudin-5 present in vesicles to reach or be spread on the barrier endothelial and/or epithelial cell membrane surface (the herein so-called Claudin-5 distribution). The effect of said peptides on Claudin-5 may be used as a marker of a positive effect of said peptides on the barrier integrity and permeability. It is also a marker of the efficacy of the peptide at treating a biological barrier dysfunction, or protecting a biological barrier.

Thus, in a first aspect, the present invention relates to a SCO-spondin-derived peptide or SCO-spondin-derived peptides for use in treating a biological barrier dysfunction, or for protecting a biological barrier.

In another aspect, the invention relates to a method for treating a biological barrier dysfunction, or for protecting a biological barrier, in a subject in need thereof, comprising administrating to said subject an effective amount of a SCO-spondin-derived peptide or of SCO-spondin-derived peptides.

In an embodiment of these different aspects of the invention, the biological barrier is the blood-brain barrier. In another embodiment, it is the blood-spinal cord barrier. In another embodiment, it is the inner blood-retina barrier. In another embodiment, it is the blood-cerebrospinal fluid barrier (BCSFB). In another embodiment, it is the lung endothelial barrier. In another embodiment, it is a vascular-parenchyma barrier. In another embodiment, it is a vascular-fluid barrier. In another embodiment, two or more of these barriers are concerned simultaneously with the use or the method of use according to the invention.

BBB impairment may be determined using several methods.

First methods comprise measurement of some markers levels in the CSF. These markers are brain-derived proteins present in the CSF such as soluble PDGFRβ (platelet-derived growth factor BB), cyclophilin A and matrix metalloproteinase-9 (Sweeney et al., Physiol Rev, 2019; Montagne et al., Nature, 2020). A BBB dysfunction is correlated with an increase of one or several of these markers.
- PGDFRβ (Montagne et al., 2020): Normal subject: 500 ng/mL, ApoE 4 carrier subject: 600 ng/mL. It is herein considered that there is dysfunction when the level is equal or above to 600 ng/mL.
- Cyclophilin A (Montagne et al., 2020): Normal subject: 60 ng/mL, ApoE 4 carrier subject: 70 ng/mL. It is herein considered that there is dysfunction when the level is equal or above to 70 ng/mL.
- MMP-9 (Montagne et al., 2020): Normal subject: 50 pg/mL, ApoE 4 carrier subject: 100 pg/mL. It is herein considered that there is dysfunction when the level is equal or above to 100 pg/mL.

Another method for determination of the dysfunction is detected by measuring regional BBB permeability constant Ktrans to the contrast tracer gadolinium identified by DCE-MRI:
- K trans in the hippocampus (Montagne et al., 2020): Normal subject: 1.2 × 10⁻³ min⁻¹ ApoE 4 carrier subject : 1.6 × 10⁻³ min⁻¹. It is considered that there is dysfunction when the level is equal or above to 1.6 × 10⁻³ min⁻¹.

Albumin quotient (Qalb) is the ratio of CSF albumin levels to serum albumin levels, Increased Qalb is the sign of BBB and BCSFB impairment. There is impairment when the patient has a QAlb > 6.5 if its age is < 40 years old and has a QAlb > 8 if its age is > 40 years old (Uher et al., 2015).

A beneficial effect of the treatment (after treatment or during treatment) of the invention on BBB impairment may be determined by measuring one or several of these markers. There is a beneficial effect of the treatment when there is a decrease of the marker level with respect to the level before treatment, or when the level measured after treatment or during treatment is close to the normal level.

Other methods may be used to characterize a BBB impairment, such as:
- increased regional BBB permeability constant, Ktrans to the contrast tracer gadolinium using dynamic contrast-enhanced magnetic resonance imaging (DCE-MRI) such as in the hippocampus in individuals with AD, or in the basal ganglia in individuals with PD (Sweeney et al., Physiol Rev, 2019; Montagne et al., Nature, 2020)
- hypointensities identified by T2* MRI or susceptibility weighted imaging (SWI MRI), indicative of microbleeds in the brains (which reflects BBB breakdown) of individuals with MCI, early AD without dementia, ALS, PD (Sweeney et al., Physiol Rev, 2019)
- reduced regional brain uptake of 2-deoxyglucose in individuals with MCI and early AD, using FDG-positron emission topography (PET) as a clinical surrogate for glucose uptake by the brain (Sweeney et al., Physiol Rev, 2019)

In an aspect, the beneficial effect on barrier integrity can be determined using one or a combination of two or more of these methods.

The beneficial effect may thus in particular be asserted by the PGDFRβ, Cyclophilin A, MMP-9, and/or Ktrans, level measurement, and observation that the peptide allows recovering the normal level or allows approaching the normal level.

In an aspect, patients that may benefit from this treatment are determined or selected before treatment. The patient is previously or has been previously determined as having a biological barrier dysfunction as described, deteriorated tight junctions as described, abnormal Claudin-5 levels as described.

In a first embodiment, the dysfunction is detected by measuring the levels soluble PDGFRβ, cyclophilin A and matrix metalloproteinase-9 (MMP-9) in the CSF.
- PGDFRβ level is equal or above to 600 ng/mL.
- Cyclophilin A level is equal or above to 70 ng/mL.
- MMP-9 level is equal or above to 100 pg/mL.

In another embodiment, the dysfunction is detected by measuring the ratio of CSF albumin levels to serum albumin levels.

In another embodiment, the dysfunction is detected by measuring regional BBB permeability constant Ktrans to the contrast tracer gadolinium identified by DCE-MRI.
- K trans level in the hippocampus is equal or above to 1.6 × 10⁻³ min⁻¹.

In another embodiment, the dysfunction is detected by measuring hypointensities identified by T2* MRI or SWI MRI.

In another embodiment, the dysfunction is detected by measuring regional brain uptake of 2-deoxyglucose using FDG-PET.

In an aspect, the dysfunction is determined using a combination of two or more of these methods.

In an aspect, the biological barrier is the blood-brain barrier (BBB) and the patient suffers from a biological barrier dysfunction in disease or conditions comprising vascular diseases, infections, immune system hyper-responsiveness, inflammatory diseases or autoimmune diseases, chronic kidney disease, intestinal diseases, macular degeneration, diabetic macular edema, epilepsy, neuropathic pain, CNS diseases and disorders, and aging.

In an aspect, the patient is first tested as recited to determine whether or not he has a BBB dysfunction. The dysfunction is determined using a combination of two or more of the above methods.

In another aspect, biological barrier dysfunction is the BBB and no disease or condition has been diagnosed in the patient.

In another aspect, the invention relates to a SCO-spondin-derived peptide or SCO-spondin-derived peptides for use in treating a biological barrier tight junction dysfunction, or for protecting said tight junctions.

In another aspect, the invention relates to a method for treating a biological barrier tight junction dysfunction, or for protecting said tight junctions, in a subject in need thereof, comprising administrating to said subject an effective amount of a SCO-spondin-derived peptide or of SCO-spondin-derived peptides.

In an embodiment of these different aspects of the invention, the biological barrier is the blood-brain barrier. In another embodiment, it is the blood-spinal cord barrier. In another embodiment, it is the inner blood-retina barrier. In another embodiment, it is the blood-cerebrospinal fluid barrier. In another embodiment, it is the lung endothelial barrier. In another embodiment, it is a vascular-parenchyma barrier. In another embodiment, it is a vascular-fluid barrier. In another embodiment, two or more of these barriers are concerned simultaneously with the use or the method of use according to the invention.

As mentioned above, a beneficial effect of the treatment on BBB impairment may be determined using the following methods:
- levels of brain-derived proteins present in the CSF such as soluble PDGFRβ (platelet-derived growth factor BB), cyclophilin A and matrix metalloproteinase-9 (Sweeney et al., Physiol Rev, 2019; Montagne et al., Nature, 2020)
- albumin quotient (Qalb), which is the ratio of CSF albumin levels to serum albumin levels, such as in individuals with preclinical AD (without clinical signs and symptoms), mild cognitive impairment (MCI), AD with or without vascular risk factors, early PD before dementia, ALS, MS (Sweeney et al., Physiol Rev, 2019; Uher et al., 2015)
- increased regional BBB permeability constant, Ktrans to the contrast tracer gadolinium using dynamic contrast-enhanced magnetic resonance imaging (DCE-MRI) such as in the hippocampus in individuals with AD, or in the basal ganglia in individuals with PD (Sweeney et al., Physiol Rev, 2019; Montagne et al., Nature, 2020)
- hypointensities identified by T2* MRI or susceptibility weighted imaging (SWI MRI), indicative of microbleeds in the brains (which reflects BBB breakdown) of individuals with MCI, early AD without dementia, ALS, PD (Sweeney et al., Physiol Rev, 2019)
- reduced regional brain uptake of 2-deoxyglucose in individuals with MCI and early AD, using FDG-positron emission topography (PET) as a clinical surrogate for glucose uptake by the brain (Sweeney et al., Physiol Rev, 2019)

In an aspect, the beneficial effect on barrier integrity can be determined using one or a combination of two or more of these methods.

In an aspect, patients that may benefit from this treatment are determined or selected before treatment. The patient is previously or has been previously determined as having a biological barrier dysfunction as described, deteriorated tight junctions as described, abnormal Claudin-5 levels as described.

In a first embodiment, the dysfunction is detected by measuring the levels soluble PDGFRβ, cyclophilin A and matrix metalloproteinase-9 in the CSF. Levels corresponding to dysfunction are mentioned above. In another embodiment, the dysfunction is detected by measuring the ratio of CSF albumin levels to serum albumin levels.

In another embodiment, the dysfunction is detected by measuring regional BBB permeability constant Ktrans to the contrast tracer gadolinium identified by DCE-MRI.

In another embodiment, the dysfunction is detected by measuring hypointensities identified by T2* MRI or SWI MRI.

In another embodiment, the dysfunction is detected by measuring regional brain uptake of 2-deoxyglucose using FDG-PET.

In an aspect, the tight junction dysfunction is determined using a combination of two or more of these methods.

In an aspect, the biological barrier is the blood-brain barrier (BBB) and the patient suffers from a central nervous system (CNS) disease, such as a neurodegenerative disease.

In an aspect, the patient is first tested as recited to determine whether or not he has a BBB dysfunction. The dysfunction is determined using a combination of two or more of the above methods.

### Detailed description

The CNS is protected by the blood-brain barrier (BBB), the blood-spinal cord barrier (BSCB), and the blood-cerebrospinal fluid barrier (BCSFB).

The BBB plays a major role in (1) regulating cerebral blood flow which is required for CNS homeostasis, (2) controlling the transport of glucose and oxygen and other metabolites from the blood to the brain, and (3) allowing the selective removal of metabolic waste from the brain via the brain vasculature (Sweeney et al, 2018). The BBB is thus critical for the proper function of neuronal circuits; therefore, BBB dysfunction or disruption has been identified as a critical component of several neurological conditions (Profaci et al., 2020). To support these functions, a physical barrier is created at the BBB structure through tight junctions between endothelial cells and the outer wall of the endothelium is then enclosed by pericytes and the astrocytic end-feet. Tight junctions ensure the paracellular selective permeability to solutes. Tight junction proteins at the BBB comprise occludins, zonulins, junctional adhesion molecules and claudins, with claudin-5 being the most expressed claudin in the brain vasculature (Profaci et al., 2020). Although endothelial cells predominantly control BBB integrity and functions, other components of the neurovascular unit (e.g. pericytes, glia and neurons) also play an important role in maintaining normal BBB functions.

The BSCB and the BBB share the same specialized system of nonfenestrated endothelial cells and their accessory structures including the basement membrane, pericytes and astrocytes (Bartanusz et al., 2011). However, the BSCB differs from the BBB in terms of levels of specific tight and adherence junction proteins, glycogen deposits and transporter molecules, which makes the BSCB more permeable than the BBB (Bartanusz et al., 2011).

The BCSFB plays a major role in (1) preventing blood-borne pathogenic components to enter the CSF, and (2) ensuring the efflux of waste products (Liddelow, 2015). The BCSFB is formed from the different types of cell junctions between the epithelial cells of the choroid plexus of brain ventricles (Solar et al., 2020). Tight junction protein families of the BCSF include occludins, zonulins, junctional adhesion molecules and claudins, similar to those present at the BBB (Solar et al., 2020).

Barrier pathology is recognized as a major contributor of the pathogenesis and progression of several neurodegenerative diseases (such as AD, ALS, PD, HD) (Kakaroubas et al., 2019; Yamazaki et al., 2019; Sweeney et al., 2018; Meister et al., 2015; Bartanusz et al., 2011), neurovascular diseases (such as ischemic stroke, AD) (Abdullahi et al., 2018; Lv et al., 2018), CNS injuries (such as SCI, TBI) (Cash and Theus, 2020; van Vliet et al., 2020; Evran et al., 2020; Bartanusz et al., 2011), psychiatric disorders (such as Schizophrenia) (Greene et al., 2018), autoimmune diseases (such as multiple sclerosis) (Berghoff et al., 2017; Bartanusz et al., 2011), eye diseases (such as macular degeneration, diabetic macular edema) (Arima et al., 2020; Hudson et al., 2019; van der Wijk et al., 2019), epilepsy (Profaci et al., 2020), neuropathic pain (Bartanusz et al., 2011), and miscellaneous conditions (such as Sars-Cov2 infections and aging) (Pellegrini et al., 2020; Buzhdygan et al., 2020).

### SCO-spondin derived peptides for use in the uses and methods disclosed herein:

"SCO-Spondin" is a glycoprotein specific to the central nervous system and present in all of the vertebrates, from prochordals to humans. It is known as a molecule of extracellular matrices that is secreted by a specific organ located in the roof of the third ventricle, the sub-commissural organ. It is a molecule of large size. It consists of more than 4,500 amino acids and has a multi-modular organization that comprises various preserved protein patterns, including in particular 26 TR or TSR patterns. It is known that certain peptides derived from SCO-Spondin starting from TSR patterns have a biological activity in the nerve or neural cells (in particular described in WO-99/03890).

"TSR or TR patterns" are protein domains of approximately 55-60 residues, based on the alignment of preserved amino acids cysteine, tryptophan and arginine. These patterns were first isolated in TSP-1 (thrombospondin 1), a molecule that intervenes in coagulation. They were then described in numerous other molecules such as SCO-Spondin. In fact, this thrombospondin type 1 unit (TSR) comprises, in all the proteins studied so far and previously mentioned, about 55- 60 amino acids (AA) some of which, like cysteine (C), tryptophan (W), serine (S), glycine (G), arginine (R) and proline (P) are highly conserved.

SCO-Spondin peptides or peptide compounds are used in performing the invention (the different objects of the invention, say peptide or composition for use, method of use, method of treatment, use of a peptide for the manufacture of a medicament, etc.). Also used are pharmaceutical compositions comprising at least one of the peptides according to the invention, and a pharmaceutically acceptable vehicle, carrier or diluent.

In particular, the invention uses a peptide of sequence
X1-W-S-A1-W-S-A2-C-S-A3-A4-C-G-X2 (SEQ ID NO: 1) in which:
A1, A2, A3 and A4 consists of amino acid sequences consisting of 1 to 5 amino acids,
the two cysteines form a disulfide bridge or not,
X1 and X2 consists of amino acid sequences consisting of 1 to 6 amino acids; or X1 and X2 are absent;
it being possible for the N-terminal amino acid to be acetylated (e.g. bears H₃CCOHN-), for the C-terminal amino acid to be amidated (e.g. bears -CONH₂), or both the N-terminal amino acid to be acetylated and the C-terminal amino acid to be amidated.

In an embodiment, in the formula of SEQ ID NO: 1, X1 or X2 or both X1 and X2 are absent. In an embodiment, where X1 and/or X2 is absent, the N-terminal W is acetylated and/or the C-terminal G is amidated. Preferably, both X1 and X2 are absent and the N-terminal W is acetylated and the C-terminal G is amidated.

In particular, the invention uses a peptide of sequence
W-S-A1-W-S-A2-C-S-A3-A4-C-G (SEQ ID NO: 2) in which:
A1, A2, A3 and A4 consists of amino acid sequences consisting of 1 to 5 amino acids,
the two cysteines form a disulfide bridge or not.

In an embodiment of the formulae of SEQ ID NO: 1 and 2, the peptide is a linear peptide, or the cysteines appearing on the peptide formula of SEQ ID NO: 1 and 2 do not form a disulfide bridge (reduced form).

In a preferred embodiment, the two cysteines appearing on the peptide formula of SEQ ID NO: 1 and 2 form a disulfide bridge (oxidized form).

Preferably, in the formulae of SEQ ID NO: 1 and 2, A1, A2, A3 and/or, preferably and A4 consist preferably of 1 or 2 amino acids, more preferably of 1 amino acid.

Preferably, A1 is chosen from G, V, S, P and A, more preferably G, S.

Preferably A2 is chosen from G, V, S, P and A, more preferably G, S.

Preferably, A3 is chosen from R, A and V, more preferably R, V.

Preferably, A4 is chosen from S, T, P and A, more preferably S, T.

Preferably, A1 and A2 are independently chosen from G and S.

Preferably, A3-A4 is chosen from R-S or V-S or V-T or R-T.

Preferably, X1, X2, A1, A2, A3 and A4 do not comprise cysteine.

When X1 is an amino acid sequence of 1 to 6 amino acids, the amino acids are any amino acid, and preferably chosen from V, L, A, P, and any combination thereof.

When X2 is an amino acid sequence of 1 to 6 amino acids, the amino acids are any amino acid, and preferably chosen from L, G, I, F, and any combination thereof.

In an embodiment, the peptide of SEQ ID NO: 1 or 2 is such that A1 and A2 are independently chosen from G and S and A3-A4 is chosen from R-S or V-S or V-T or R-T.

In a particular modality, this peptide is further acetylated and/or amidated. In an embodiment, the peptide is a linear peptide, or the cysteines do not form a disulfide bridge. In another embodiment, the peptide has the two cysteines forming a disulfide bridge (C-terminal cyclization). In another embodiment, the peptide as used in the invention or the peptide administered to the patient does comprise both forms, oxidized peptide and linear peptide.

For the purposes of the present invention, the term "amino acids" means both natural amino acids and non-natural amino acids and changes of amino acids, including from natural to non-natural, may be made routinely by the skilled person while keeping the function or efficacy of the original peptide. By "natural amino acids" is meant the amino acids in L form that may be found in natural proteins, *i.e.* alanine, arginine, asparagine, aspartic acid, cysteine; glutamine, glutamic acid, glycine, histidine, isoleucine, leucine, lysine, methionine, phenylalanine, proline, serine, threonine, tryptophan, tyrosine and valine. By "non-natural amino acid" is meant the preceding amino acids in their D form, as well as the homo forms of certain amino acids such as arginine, lysine, phenylalanine and serine, or the nor forms of leucine or valine. This definition also comprises other amino acids such as alpha-aminobutyric acid, agmatine, alpha-aminoisobutyric acid, sarcosine, statin, ornithine, deaminotyrosine. The nomenclature used to describe the peptide sequences is the international nomenclature using the one-letter code and where the amino-terminal end is shown on the left and the carboxy-terminus is shown on the right. The dashes "-" represent common peptide bonds linking the amino acids of the sequences.

In an embodiment, the peptide according to the invention, for example any one of the peptides of sequence SEQ ID NO: 1-63, comprises an N-terminal acetylation, a C-terminal amidation, or both an N-terminal acetylation and a C-terminal amidation.

In different embodiments, the invention relates to the use of polypeptides consisting essentially of, or consisting of the following amino acid sequences (Table A):

| | |
|---|---|
| W-S-G-W-S-S-C-S-R-S-C-G | SEQ ID NO : 3 |
| W-S-S-W-S-G-C-S-R-S-C-G | SEQ ID NO : 4 |
| W-S-S-W-G-S-C-S-R-S-C-G | SEQ ID NO : 5 |
| W-S-S-W-G-G-C-S-R-S-C-G | SEQ ID NO : 6 |
| W-S-S-W-S-S-C-S-R-S-C-G | SEQ ID NO : 7 |
| W-S-G-W-S-G-C-S-R-S-C-G | SEQ ID NO : 8 |
| W-S-G-W-G-S-C-S-R-S-C-G | SEQ ID NO : 9 |
| W-S-G-W-G-G-C-S-R-S-C-G | SEQ ID NO : 10 |
| W-S-S-W-S-S-C-S-V-S-C-G | SEQ ID NO : 11 |
| W-S-S-W-S-G-C-S-V-S-C-G | SEQ ID NO : 12 |
| W-S-S-W-G-S-C-S-V-S-C-G | SEQ ID NO : 13 |
| W-S-S-W-G-G-C-S-V-S-C-G | SEQ ID NO : 14 |
| W-S-G-W-S-S-C-S-V-S-C-G | SEQ ID NO : 15 |
| W-S-G-W-S-G-C-S-V-S-C-G | SEQ ID NO : 16 |
| W-S-G-W-G-S-C-S-V-S-C-G | SEQ ID NO : 17 |
| W-S-G-W-G-G-C-S-V- | SEQ ID NO : 18 |
| W-S-S-W-S-S-C-S-V-T-C-G | SEQ ID NO : 19 |
| W-S-S-W-S-G-C-S-V-T-C-G | SEQ ID NO : 20 |
| W-S-S-W-G-S-C-S-V-T-C-G | SEQ ID NO : 21 |
| W-S-S-W-G-G-C-S-V-T-C-G | SEQ ID NO : 22 |
| W-S-G-W-S-S-C-S-V-T-C-G | SEQ ID NO : 23 |
| W-S-G-W-S-G-C-S-V-T-C-G | SEQ ID NO : 24 |
| W-S-G-W-G-S-C-S-V-T-C-G | SEQ ID NO : 25 |
| W-S-G-W-G-G-C-S-V-T-C-G | SEQ ID NO : 26 |
| W-S-S-W-S-S-C-S-R-T-C-G | SEQ ID NO : 27 |
| W-S-S-W-S-G-C-S-R-T-C-G | SEQ ID NO : 28 |
| W-S-S-W-G-S-C-S-R-T-C-G | SEQ ID NO : 29 |
| W-S-S-W-G-G-C-S-R-T-C-G | SEQ ID NO : 30 |
| W-S-G-W-S-S-C-S-R-T-C-G | SEQ ID NO : 31 |
| W-S-G-W-S-G-C-S-R-T-C-G | SEQ ID NO : 32 |
| W-S-G-W-G-S-C-S-R-T-C-G | SEQ ID NO : 33 |
| W-S-G-W-G-G-C-S-R-T-C-G | SEQ ID NO : 34 |

In an embodiment, the peptides of sequences SEQ ID NO: 3-34 disclosed in Table A are linear peptides, or the cysteines do not form a disulfide bridge (reduced peptides). In another embodiment, the peptides of sequences SEQ ID NO: 3-34 disclosed in the preceding Table A have the two cysteines oxidized to form a disulfide bridge (oxidized peptides). In another embodiment, the peptides as used in the invention or the peptides administered to the patient do comprise both forms, oxidized peptide and linear peptide of the same peptide sequence. In still another embodiment, the peptides as used in the invention or the peptides administered to the patient do comprise a mixture of at least two of these different peptides chosen from sequences SEQ ID NO: 3-34, wherein the mixture may be a mixture of at least two linear peptides or a mixture of at least two oxidized peptides, or a mixture of at least one linear peptide and at least one oxidized peptide, for example having the same amino acid sequence.

In a preferred embodiment, the peptide consists of the amino acid sequence W-S-G-W-S-S-C-S-R-S-C-G (SEQ ID NO: 3). In an embodiment, the peptide is a linear peptide, or the cysteines do not form a disulfide bridge (reduced form called NX210). In another embodiment, the peptides have the two cysteines oxidized to form a disulfide bridge (oxidized form), it is called NX218. In another embodiment, the peptides as used in the invention or the peptides administered to the patient do comprise both forms, oxidized and reduced.

In an embodiment of the peptides of SEQ ID NO: 1,
- X1 represents a hydrogen atom or P or A-P or L-A-P or V-L-A-P, and/or
- X2 represents a hydrogen atom or L or L-G or L-G-L or L-G-L-I or L-G-L-I-F.

In different embodiments, the invention thus relates to the use of polypeptides consisting or consisting essentially of the following amino acid sequences (Table B):

| | |
|---|---|
| P-W-S-G-W-S-S-C-S-R-S-C-G | SEQ ID NO : 35 |
| A-P-W-S-G-W-S-S-C-S-R-S-C-G | SEQ ID NO : 36 |
| L-A-P-W-S-G-W-S-S-C-S-R-S-C-G | SEQ ID NO : 37 |
| V-L-A-P-W-S-G-W-S-S-C-S-R-S-C-G | SEQ ID NO : 38 |
| W-S-G-W-S-S-C-S-R-S-C-G-L | SEQ ID NO : 39 |
| W-S-G-W-S-S-C-S-R-S-C-G-L-G | SEQ ID NO : 40 |

| | |
|---|---|
| W-S-G-W-S-S-C-S-R-S-C-G-L-G-L | SEQ ID NO : 41 |
| W-S-G-W-S-S-C-S-R-S-C-G-L-G-L-I | SEQ ID NO : 42 |
| W-S-G-W-S-S-C-S-R-S-C-G-L-G-L-I-F | SEQ ID NO : 43 |
| P-W-S-G-W-S-S-C-S-R-S-C-G-L | SEQ ID NO : 44 |
| P-W-S-G-W-S-S-C-S-R-S-C-G-L-G | SEQ ID NO : 45 |
| P-W-S-G-W-S-S-C-S-R-S-C-G-L-G-L | SEQ ID NO : 46 |
| P-W-S-G-W-S-S-C-S-R-S-C-G-L-G-L-I | SEQ ID NO : 47 |
| P-W-S-G-W-S-S-C-S-R-S-C-G-L-G-L-I-F | SEQ ID NO : 48 |
| A-P-W-S-G-W-S-S-C-S-R-S-C-G-L | SEQ ID NO : 49 |
| A-P-W-S-G-W-S-S-C-S-R-S-C-G-L-G | SEQ ID NO : 50 |
| A-P-W-S-G-W-S-S-C-S-R-S-C-G-L-G-L | SEQ ID NO : 51 |
| A-P-W-S-G-W-S-S-C-S-R-S-C-G-L-G-L-I | SEQ ID NO : 52 |
| A-P-W-S-G-W-S-S-C-S-R-S-C-G-L-G-L-I-F | SEQ ID NO : 53 |
| L-A-P-W-S-G-W-S-S-C-S-R-S-C-G-L | SEQ ID NO : 54 |
| L-A-P-W-S-G-W-S-S-C-S-R-S-C-G-L-G | SEQ ID NO : 55 |
| L-A-P-W-S-G-W-S-S-C-S-R-S-C-G-L-G-L | SEQ ID NO : 56 |
| L-A-P-W-S-G-W-S-S-C-S-R-S-C-G-L-G-L-I | SEQ ID NO : 57 |
| L-A-P-W-S-G-W-S-S-C-S-R-S-C-G-L-G-L-I-F | SEQ ID NO : 58 |
| V-L-A-P-W-S-G-W-S-S-C-S-R-S-C-G-L | SEQ ID NO : 59 |
| V-L-A-P-W-S-G-W-S-S-C-S-R-S-C-G-L-G | SEQ ID NO : 60 |
| V-L-A-P-W-S-G-W-S-S-C-S-R-S-C-G-L-G-L | SEQ ID NO : 61 |
| V-L-A-P-W-S-G-W-S-S-C-S-R-S-C-G-L-G-L-I | SEQ ID NO : 62 |
| V-L-A-P-W-S-G-W-S-S-C-S-R-S-C-G-L-G-L-I-F | SEQ ID NO : 63 |

In an embodiment, the peptides of sequences SEQ ID NO: 35-63 disclosed in Table B, or of sequences SEQ ID NO: 3-63 disclosed in Tables A + B, are linear peptides, or the cysteines do not form a disulfide bridge (reduced peptides). In another embodiment, the peptides have the two cysteines oxidized to form a disulfide bridge (oxidized peptides). In another embodiment, the peptides as used in the invention or the peptides administered to the patient do comprise both forms, oxidized peptide and linear peptide of the same peptide sequence. In still another embodiment, the peptides as used in the invention or the peptides administered to the patient do comprise a mixture of at least two of these different peptides chosen from sequences SEQ ID NO: 35-63, or 3-63, wherein the mixture may be a mixture of at least two linear peptides or a mixture of at least two oxidized peptides, or a mixture of at least one linear peptide and at least one oxidized peptide, for example having the same amino acid sequence.

Each one of the peptides of sequences SEQ ID NO: 3-63 may be acetylated, amidated, or acetylated and amidated.

The peptides as used in the invention or the peptides administered to the patient are defined with their amino acid sequences. The peptides as used may be one peptide as disclosed herein, or a mixture of at least two peptides as disclosed herein. The mixtures also encompass the mixture of linear and oxidized peptides, of the same or different amino acid sequences. If a 100% pure peptide may be used, in accordance with the invention, it is possible, and the invention encompasses, that the peptide has a purity greater than 80%, preferably 85%, more preferably 90%, even more preferably equal to or greater than 95, 96, 97, 98, or 99%. Conventional purification methods, for example by chromatography, may be used to purify the desired peptide compound.

In an embodiment, the peptide as used in the invention or the peptide administered to the patient do comprise both forms, oxidized peptide (Op) and linear peptide (Lp), for instance in similar amounts or not, e.g. (% in number) Op: 10, 20, 25, 30, 40, 50, 60, 70, 80, or 90 %, the remaining to 100% being the Lp. The oxidized peptide and the linear peptide that are combined may be of the same sequence or of different sequences. For example, the oxidized and linear forms of the peptide of sequence SEQ ID NO: 3 are so combined (NX210 and NX218), for example in the proportions disclosed above. The same apply to any one of the peptides of sequence SEQ ID NO: 4-34 and 35-63.

### Pharmaceutical compositions:

The pharmaceutical compositions as used herein comprise as active ingredient a peptide or mixture of peptides as previously described, for example peptides of different amino acid composition or peptides of the same amino acid composition under oxidized and linear forms, and one or more pharmaceutically-acceptable vehicles, carriers or excipients.

The peptide compounds according to the invention may be used in a pharmaceutical composition, or in the manufacture of a medicament for preventing or treating any one of the body dysfunction disclosed herein, such as a biological barrier dysfunction and/or deteriorated tight junctions.

In a first embodiment, the dysfunction is detected by measuring the levels soluble PDGFRβ in the CSF.

In another embodiment, the dysfunction is detected by measuring the ratio of CSF albumin levels to serum albumin levels.

In another embodiment, the dysfunction is detected by measuring regional BBB permeability constant Ktrans to the contrast tracer gadolinium identified by DCE-MRI.

In another embodiment, the dysfunction is detected by measuring hypointensities identified by T2* MRI or SWI MRI.

In another embodiment, the dysfunction is detected by measuring regional brain uptake of 2-deoxyglucose using FDG-PET.

In these compositions or medicaments, the active principle may be incorporated into compositions in various forms, *i.e.* in the form of solutions, generally aqueous solutions, or in freeze-dried form, or in the form of emulsion or any other pharmaceutically and physiologically acceptable form suited to the administration route.

Administration route may be a systemic route. Mention may be made in particular of the following injection or administration routes: intravenous, intrathecal, intraperitoneal, intranasal, subcutaneous, intramuscular, sublingual, oral, and combinations thereof.

Administration may also be local notably using intracerebral routes, especially intracerebroventricular administration.

The compositions containing one or more of the herein-disclosed peptides are sterile. These compositions are suitable for an administration leading to delivering the peptide(s) into the patient, e.g. in blood circulation. Delivery to the patient is delivery of a sufficient amount of the peptide(s), and this sufficient amount is correlated with the beneficial effect. The "pharmaceutical effect" may comprise one or several of the beneficial effects on BBB, the tight junctions, the Claudin-5 distribution, the transendothelial and/or transepithelial electrical resistance, the trans-compartment permeability deregulation of specific compounds or molecules, as disclosed herein.

In some embodiments, the active principle in the pharmaceutical composition consists of (1) a linear peptide as disclosed herein, (2) an oxidized peptide as disclosed herein, (3) NX210, (4) NX218 or (5) a mixture of linear and oxidized peptides, such as in particular NX210 and NX218, in similar amounts or not, as disclosed above.

The active principle can be administered in a unit administration form, as a mixture with conventional pharmaceutical supports, carriers, excipients or vehicles, to animals and human beings. Suitable unit administration forms comprise oral-route forms such as tablets, gel capsules, powders, granules and oral suspensions or solutions, sublingual and buccal administration forms, aerosols; implants; subcutaneous, transdermal, topical, intraperitoneal, intramuscular, intravenous, subdermal, transdermal and intranasal administration forms and rectal administration forms.

Preferably, the pharmaceutical compositions contain carriers, excipients or vehicles which are pharmaceutically acceptable for a liquid formulation capable of being administered, e.g. injected to deliver the active principle in the patient, e.g. in blood stream. These may be in particular ready to use solutions, such as isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of administrable solutions.

The pharmaceutical forms include sterile aqueous solutions or dispersions; formulations including sesame oil, peanut oil or aqueous propylene glycol; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi.

Sterile injectable solutions are prepared by incorporating the active polypeptides in the required amount in the appropriate solvent followed by filtered sterilization.

Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed.

For suitable administration in an aqueous solution, for example, the solution should be suitably buffered if necessary and the liquid diluent first rendered isotonic with sufficient saline or glucose. These particular aqueous solutions are especially suitable for administration, e.g. intravenous, intramuscular, subcutaneous and intraperitoneal administration. In this connection, sterile aqueous media which can be employed will be known to those of skill in the art in light of the present disclosure. In addition to the compounds of the invention formulated for injection administration, such as intravenous or intramuscular injection, other pharmaceutically acceptable forms include, e.g. tablets or other solids for oral administration; liposomal formulations; time release capsules; and any other form currently used, and delivering the active principle.

One dose of peptide(s) is expressed in weight of peptide per patient body weight (kg) and may range from about 1 µg/kg to about 1 g/kg, in particular from about 10 µg/kg to about 100 mg/kg, e.g. from about 50 µg/kg to about 50 mg/kg.

The dosage regimen may comprise a single administration or repeated administrations. According to an embodiment, repeated administrations may comprise administering one dose per day of treatment, for example one dose every day or every 2 or 3 days over a treatment period. According to another embodiment, repeated administrations may comprise administering at least two doses per day of treatment, for example 2, 3 or more doses per day over a treatment period. In these embodiments, a treatment period may be 1, 2, 3, 4, 5, 6, 7, 8, 9, 10, 15, 20, 25, 30, 35, 40, 45 or more days (e.g. up to 6 months). The treatment is designed so that the patient keeps "benefit" from this treatment over a "period of time". "Benefit" may comprise the "pharmaceutical effect" mentioned above, say the peptides are beneficial to the subject suffering or in risk of suffering of one of these diseases. The peptides thus allow for an improvement (increase, reestablishing or protecting) of the tight junctions of epithelial and/or endothelial cells involved in a biological barrier, of the paracellular permeability at the level of these cells or barriers, and/or of the Claudin-5 distribution (an increase of Claudin-5 level at the surface of the barrier endothelial and/or epithelial cells, especially they cause Claudin-5 present in vesicles to reach or be spread on the barrier endothelial and/or epithelial cell membrane surface) and thus an improvement of the functional biological barriers, such as the BBB, and of associated diseases. This "period of time" may depend from the dosage regimen and from the patient itself, e.g. the severity of the illness and the responsiveness of the patient to the regimen dose. "Improvement" comprises "partial improvement" and "total improvement". It is said "partial" when in the subject it is observed a partial recovery of the biological barrier integrity or the tight junctions integrity, and of the physiological functions thereof; with respect to the initial status of the subject before treatment, there is a significant improvement of these, however it remains significantly below with respect to healthy subjects. "Total recovery" means that the subject recovered the biological barrier integrity or the tight junctions integrity, and of the physiological functions thereof; or that they are not significantly different than healthy subjects. As explained, the integrity of the barrier or the recovery of it may be assessed using the methods disclosed herein.

The administration regimen may be suited to the severity of the barrier dysfunction. In case of mild severity, the regimen may comprise administration every three days. In case of moderate severity, the regimen may comprise administration every two other days. In case of severe severity, the regimen may comprise administration everyday.

In case of no disease or condition known to be related to the biological barrier dysfunction, the regimen may be every three days or every two other days.

### Further Definitions:

Administration or use of "peptide" or "peptides" or "peptide(s)", is a generic wording, and the invention encompasses administration or use of one single peptide or more than one single peptide, *i.e.* the administration or use of at least two peptides according to the present disclosure. Thus, in the present disclosure the singular or the plural is not limited unless indicated to the contrary, and may each time encompass one single peptide, or at least two peptides. The same apply to the equivalent wording "peptide compound" that may be used interchangeably for "peptide".

"Treating", "treated", or "treat", means delivering an amount of peptide compound according to the invention to a subject. These terms as used herein refers to a therapeutic wherein the object is to slow down (lessen) an undesired physiological condition, disorder or disease, or to obtain beneficial or desired clinical results. For the purposes of this invention, beneficial or desired clinical results include, but are not limited to, modulating, stabilizing, correcting a body dysfunction, such as a biological barrier dysfunction or a tight junctions dysfunction; this may involve modulating, stabilizing, correcting, increasing, lowering, one or more markers linked thereto, such as the Claudin-5 level or distribution. For the purposes of this invention, beneficial or desired clinical results also include stabilization (*i.e.* not worsening) of the state of the symptoms, condition, disorder or disease; delay in onset or slowing of the progression of the symptoms, condition, disorder or disease; amelioration of the symptoms, condition, disorder or disease state; and remission (whether partial or total), with substantial reestablishment of a normal function, or enhancement or improvement of the condition, disorder or disease. The terms "treating", "treated" or "treat" may include preventing, suppressing, repressing, ameliorating, or completely eliminating the body dysfunction or the attached possible symptoms. Preventing the disease may involve administering a composition of the present invention to a subject prior to onset of the body dysfunction or symptoms attached thereto. Suppressing these disease symptoms may involve administering a composition of the present invention to a subject after induction of the disease but before its clinical appearance. Repressing or ameliorating these disease symptoms may involve administering a composition of the present invention to a subject after clinical appearance of these disease symptoms.

"Effective amount", "sufficient amount", and similar such as "therapeutically effective amount", are used interchangeably herein unless otherwise defined, and means a dosage of a peptide or peptides of the invention effective for periods of time necessary to achieve the desired therapeutic result. An effective dosage may be determined by a person skilled in the art and may vary according to factors such as the disease state, age, sex, and weight of the individual, and the ability of the drug to elicit a desired response in the individual. This term as used herein may also refer to an amount effective at bringing about a desired in vivo effect in a subject, in particular a stabilization or a recovery of the biological barrier. A therapeutically effective amount may be administered in one or more administrations (e.g., the composition may be given as a preventative treatment or therapeutically at any stage of disease progression, before or after symptoms, and the like), applications, or dosages, and is not intended to be limited to a particular formulation, combination, or administration route. It is within the scope of the present disclosure that the peptide(s) may be administered at various times during the course of treatment of the subject. The times of administration and dosages used will depend on several factors, such as the goal of treatment (e.g., treating *vs*. preventing), condition of the subject, etc., and can be readily determined by one skilled in the art. A therapeutically effective amount is also one in which any toxic or detrimental effects of substance are outweighed by the therapeutically beneficial effects. A "prophylactically effective amount" refers to an amount effective, at dosages and for periods of time necessary, to achieve the desired prophylactic result. Typically, since a prophylactic dose is used in subjects prior to or at an earlier stage of disease, the prophylactically effective amount may be less than the therapeutically effective amount. "Effective amount", "sufficient amount", may also take into account the combination of different peptides if one considers the amount of the peptides separately, and/or the combination with another active principle, owing to which, for example, the dose of one or the two drugs in the combination may be lowered by result of a combined effect or a synergic effect.

The terms "comprise(s)," "include(s)," "having," "has," "can," "contain(s)," and variants thereof, as used herein, are intended to be open-ended transitional phrases, terms, or words that do not preclude the possibility of additional acts or products, such as peptides, compounds or drugs. The singular forms "a," "and" and "the" include plural references unless the context clearly dictates otherwise. The present disclosure also contemplates other embodiments "comprising," "consisting of" and "consisting essentially of," the embodiments or elements presented herein, whether explicitly set forth or not.

"Patient or subject" means an animal, especially a mammal, including a human. In an embodiment, the subject is a human. In other embodiments, the subject is a big or a farm animal, a companion animal (*e.g.* cat, dog) or a sport animal (*e.g.* horse).

In an embodiment of the present use or method of treatment, may be excluded one or several of the following diseases: Parkinson's Disease (PD), Multiple Sclerosis (MS), Myopathies, non-brain nervous system injury, such as Spinal Cord Injury (SCI) or Optic Nerve Injury (ONI), tauopathies (i.e. Tau positives neurodegenerative diseases, including any of Alzheimer's Disease (AD), Progressive Supranuclear Palsy (PSP), Tau positive Fronto-Temporal Dementia such as Pick's disease, dementia with Lewy bodies, corticobasal degeneration, Niemann-Pick type C disease, chronic traumatic encephalopathy including dementia pugilistica, postencephalitic parkinsonism); may be excluded individually Alzheimer's Disease (AD); Progressive Supranuclear Palsy (PSP); Tau positive Fronto-Temporal Dementia such as Pick's disease; dementia with Lewy bodies; corticobasal degeneration; Niemann-Pick type C disease; chronic traumatic encephalopathy including dementia pugilistica; postencephalitic parkinsonism; cerebral ischemia; CNS neuronal traumas (i.e spinal cord or brain injuries) pathologies; viral neurodegenerations; Amyotrophic Lateral Sclerosis; Spinal Muscular Atrophy; Huntington's Disease; prion disease; PSP; multiple system atrophy; adrenoleukodystrophy; Down syndrome.

The present invention will now be described in more details using non-limiting examples referring to the figures:

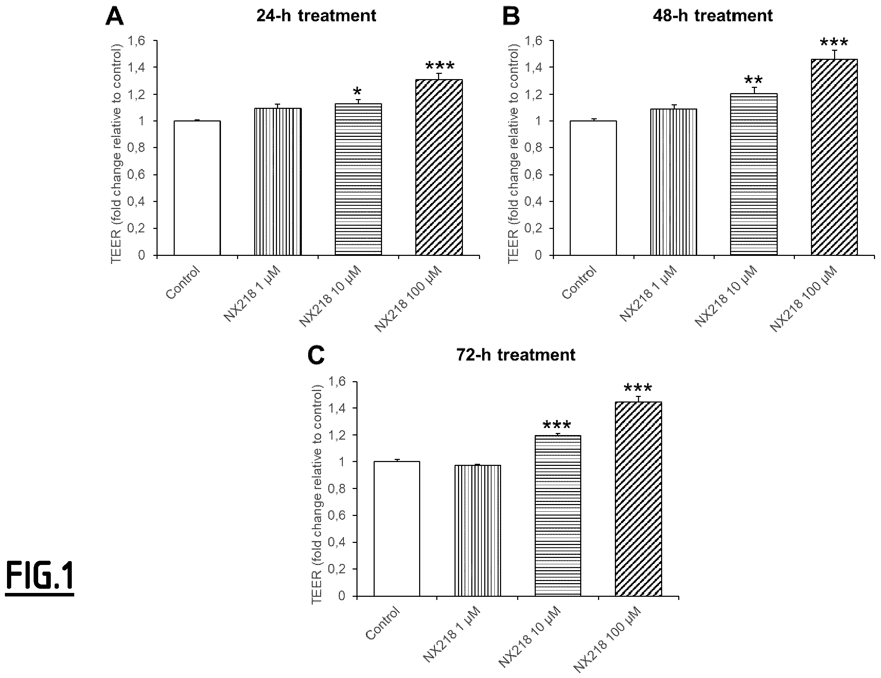

**Figure 1****: NX218 increases the transendothelial electrical resistance of mouse brain endothelial cell monolayer in a dose-dependent manner *in vitro***. The transendothelial electrical resistance (TEER) of mouse brain endothelial bEnd.3 cells grown on Transwell inserts was measured after exposure to vehicle or different doses of NX218 (1, 10, 100 µM) for 24 h (A), 48 h (B) or 72 h (C). One-way ANOVA followed by Dunnett's multiple comparisons tests: ***p < 0.0001, **p < 0.001, *p < 0.05 compared with the control group, n=6/group (A) and n=9/group (B, C).

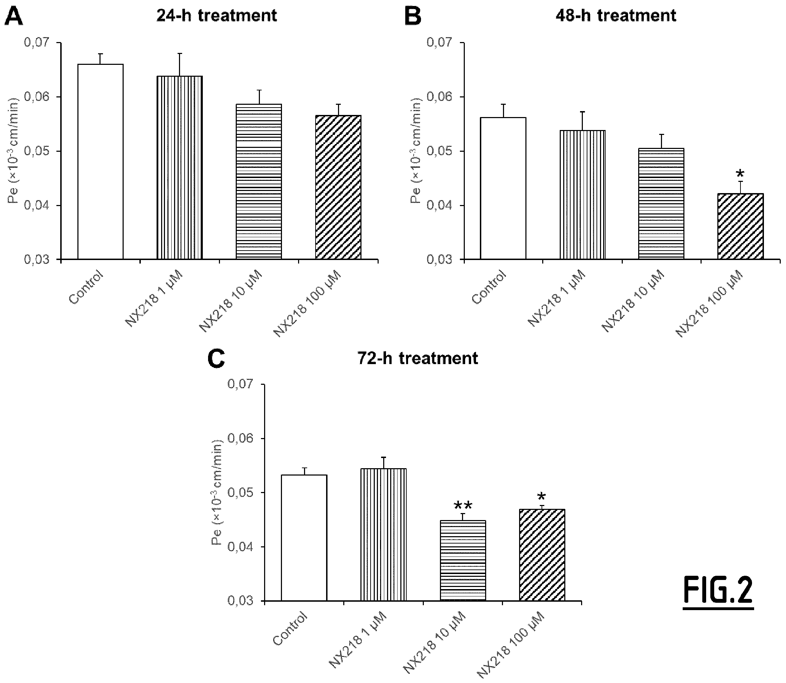

**Figure 2****: NX218 reduces the permeability of mouse brain endothelial cell monoloyer in a dose-dependent manner *in vitro.*** The permeability of mouse brain endothelial bEnd.3 cells grown on Transwell inserts to FITC-40 kDa Dextran was measured after exposure to vehicle or different doses of NX218 (1, 10, 100 µM) for 24 h (A), 48 h (B) or 72 h (C). One-way ANOVA followed by Dunnett's multiple comparisons tests: **p < 0.001, *p < 0.05 compared with the control group, n=3/group.

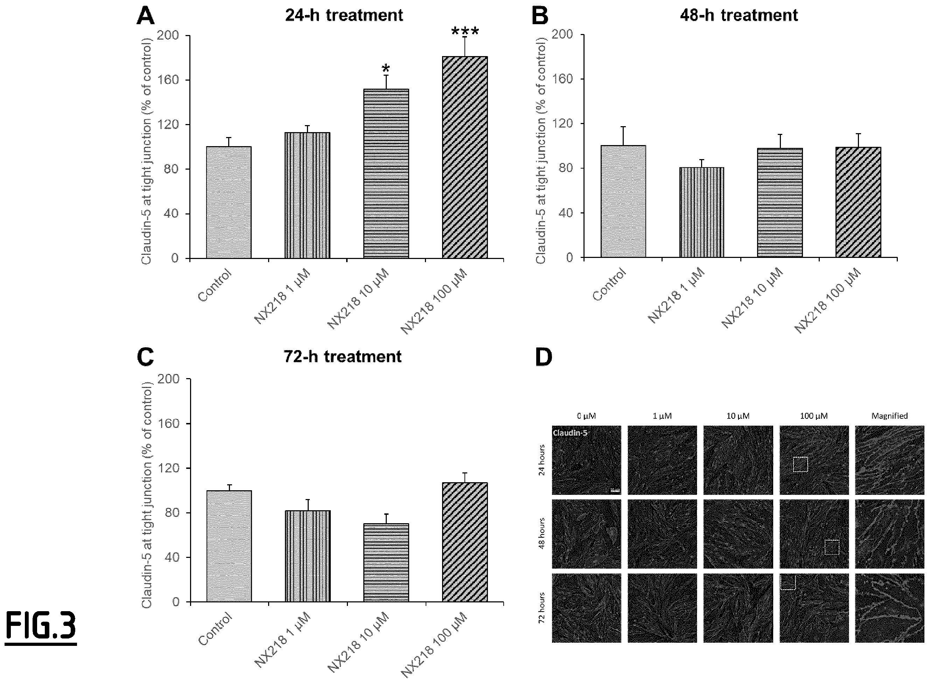

**Figure 3****: 24-hour treatment with NX218 increases the expression of Claudin-5 at tight junction in mouse brain endothelial cell cultures.** Mouse brain endothelial bEnd.3 cells were exposed to vehicle or different doses of NX218 (1, 10, 100 µM) for 24 h (A), 48 h (B) or 72 h (C), and then fixed and stained with Claudin-5. Claudin-5 immunoreactivity at membrane localization was finally quantified to evaluate Claudin-5 protein levels at tight junctions. One-way ANOVA followed by Dunnett's multiple comparisons tests: ***p < 0.0001, *p < 0.05 compared with the control group, n=6/group (A, B) and n=4/group (C). Representative photomicrographs of endothelial cells immunostained with Claudin-5 (red) (D; scale bar: 50 µm).

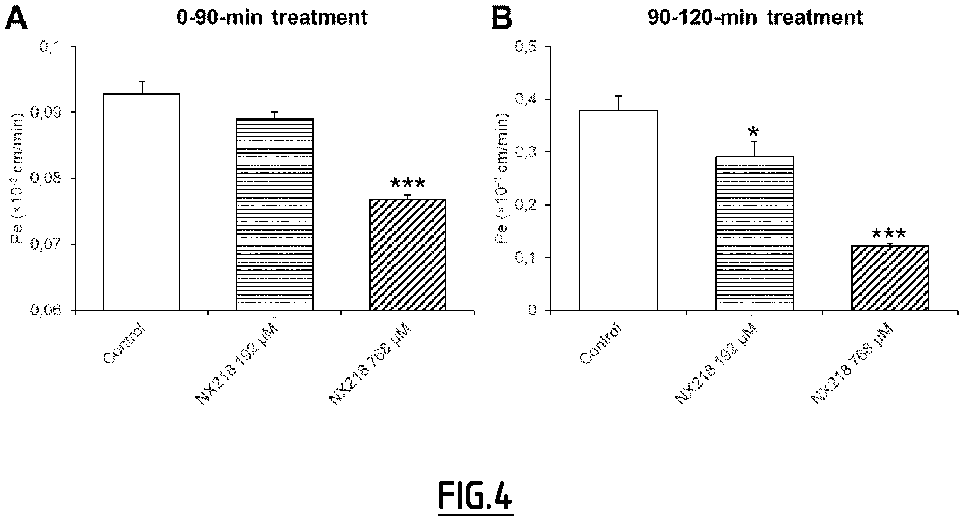

**Figure 4****: NX218 reduces the permeability of primary rat choroid plexus epithelial cell monoloyer in a dose-dependent manner in vitro.** The permeability of primary rat choroid plexus epithelial cells grown on Transwell inserts to [14C]-sucrose was measured after exposure to vehicle or different doses of NX218 (192, 768 µM) for 2 hours - 0-90 min (A), 90-120 min (B). One-way ANOVA followed by Dunnett's multiple comparisons tests: ***p < 0.0001, *p < 0.05 compared with the control group, n=4/group.

### Example 1: Synthesis of NX peptides

The manufacturing process of the peptides of sequence SEQ ID NO: 1, 2, or of any of the sequences 3-63, and especially those used in the Part Example, such as NX210 (SEQ ID NO: 3), is based on Solid-Phase Peptide Synthesis applying N-α-Fmoc (side chain) protected amino acids as building blocks in the assembly of the peptide. The protocol employed consists of a coupling of the C-terminal Glycine N-α-Fmoc-protected amino acid bound to an MPPA linker on the MBHA resin, followed by Fmoc coupling / deprotection sequences. After assembly of the peptide on the resin, a step of simultaneous cleavage of the peptide from the resin and deprotection of the side chains of amino acid is carried-out.

The crude peptide is precipitated, filtered and dried. Prior to purification by preparative reverse phase chromatography, the peptide is dissolved in an aqueous solution containing acetonitrile. The purified peptide in solution is the concentrated before undergoing an ion exchange step to obtain the peptide in the form of its acetate salt.

The skilled person may refer for further detail of synthesis to US 6,995,140 and WO2018146283, and for the oxidized forms of the peptides disclosed herein, to WO 2017/051135, all incorporated herein by reference.

The skilled person further has access to the standard methods to produce any of the disclosed peptides of the invention including the N-ter and C-ter modified or protected peptides. Concerning the acetylation and/or the amidation of the peptides at the N-terminal and C-terminal respectively, the skilled person may refer to standard techniques, e.g. those described in Biophysical Journal Volume 95 November 2008 4879-4889, also incorporated by reference.

### EXAMPLE 2: Synthesis of cyclic NX peptides

The polypeptide of sequence W-S-G-W-S-S-C-S-R-S-C-G was added to Human Serum Albumin (HSA) in a 1:1 ratio and incubated for 1 to 3 hours with stirring in air at room temperature. By using HPLC, we observed the formation of a peak corresponding to the polypeptide sequence W-S-G-W-S-S-C-S-R-S-C-G in which the 2 cysteines are linked by a disulfide bridge. After removal of the albumin by precipitation, the product was then purified and analyzed by HPLC. The use of a different ratio of albumin and of polypeptide corresponding to the sequence W-S-G-W-S-S-C-S-R-S-C-G makes it possible to influence the cyclization rate and the final yield of cyclisation, while knowing that a smaller amount of albumin is easier to eliminate. Cyclized compound is NX218.

The skilled person may refer for further detail of synthesis to WO2017051135. This document is incorporated herein by reference.

### EXAMPLE 3: NX218 effect on the transendothelial electrical resistance of mouse brain endothelial cell monolayer

To determine the effect of NX218 on transendothelial electrical resistance (TEER) of mouse brain endothelial cells. Cells were grown on a semi-permeable membrane inserted in culture wells in presence or absence of NX218 and the TEER was recorded using electrodes placed on each side of the membrane and a resistance meter.

### Cell culture:

Mouse brain endothelial cells (bEnd.3, American Type Culture Collection) were cultured in Dulbecco's modified Eagle's Medium (DMEM) supplemented with 10% fetal bovine serum (FBS) and 2 mM sodium pyruvate in a 5% CO₂ incubator at 37°C.

### TEER measurement:

bEnd.3 cells were grown on 6.5-mm-diameter, 0.4-µm-pore, polyester membrane HTS Transwell inserts (5 × 10⁴ cells per well containing 200 µL and 800 µL of culture medium in the apical and basolateral compartments respectively). Three days after seeding, cells were treated with vehicle or NX218 (1, 10 and 100 µM) for 1, 2 or 3 days. TEER values were measured using an EVOM resistance meter fitted with "chopstick" electrodes. One electrode was placed in the apical chamber and another one in the basolateral chamber. Before measurements, chambers were replaced with fresh medium.

### Results:

The results are presented in Figure 1 and in Tables 1, 2 and 3 below.

**Table 1: TEER change relative to control of mouse brain endothelial cell monolayer after exposure to vehicle or different doses of NX218 (1, 10, 100 µM) for 24 hours**

| | **Control** | **NX218 1 µM** | **NX218 10 µM** | **NX218 100 µM** |
|---|---|---|---|---|
| N1 | 0,99360614 | 1,051150895 | 1,177749361 | 1,453964194 |
| N2 | 1,00895141 | 1,196930946 | 1,212276215 | 1,373401535 |
| N3 | 0,99744246 | 1,139386189 | 1,212276215 | 1,369565217 |
| N4 | 1,03754266 | 1,09556314 | 1,027303754 | 1,218430034 |
| N5 | 0,97610922 | 1,109215017 | 1,068259386 | 1,283276451 |
| N6 | 0,98634812 | 0,976109215 | 1,071672355 | 1,141638225 |
| **Average** | **1** | **1,0947259** | **1,128256214** | **1,306712609** |
| *SEM* | *0,0087448* | *0,030886754* | *0,033446995* | *0,046788001* |

**Table 2: TEER change relative to control of mouse brain endothelial cell monolayer after exposure to vehicle or different doses of NX218 (1, 10, 100 uM) for 48 hours**

| | **Control** | **NX218 1 µM** | **NX218 10 µM** | **NX218 100 µM** |
|---|---|---|---|---|
| N1 | 0,968253968 | 0,984126984 | 1,28042328 | 1,724867725 |
| N2 | 1,026455026 | 1,195767196 | 1,380952381 | 1,71957672 |
| N3 | 1,005291005 | 1,010582011 | 1,312169312 | 1,571428571 |
| N4 | 0,972640219 | 1,149110807 | 1,218878249 | 1,547195622 |
| N5 | 1,099863201 | 1,218878249 | 1,33378933 | 1,403556772 |
| N6 | 0,92749658 | 1,194254446 | 1,218878249 | 1,497948016 |
| N7 | 1,015642458 | 0,988826816 | 1,015642458 | 1,223463687 |
| N8 | 0,982122905 | 1,005586592 | 1,052513966 | 1,283798883 |
| N9 | 1,002234637 | 1,055865922 | 1,015642458 | 1,173184358 |
| **Average** | **1** | **1,089222114** | **1,203209965** | **1,460557817** |
| *SEM* | *0,01593516* | *0,03295557* | *0,04714646* | *0,067847557* |

**Table 3: TEER change relative to control of mouse brain endothelial cell monolayer after exposure to vehicle or different doses of NX218 (1, 10, 100 uM) for 72 hours**

| | **Control** | **NX218 1 µM** | **NX218 10 µM** | **NX218 100 µM** |
|---|---|---|---|---|
| N1 | 1,04109589 | 0,931506849 | 1,150684932 | 1,237442922 |
| N2 | 0,940639269 | 0,940639269 | 1,146118721 | 1,305936073 |
| N3 | 1,01826484 | 0,945205479 | 1,146118721 | 1,347031963 |
| N4 | 0,971332209 | 0,996627319 | 1,269814503 | 1,467116358 |
| N5 | 0,966273187 | 1,021922428 | 1,254637437 | 1,55311973 |
| N6 | 1,062394604 | 1,006745363 | 1,239460371 | 1,623946037 |
| N7 | 0,932088285 | 1,003395586 | 1,186757216 | 1,436332767 |
| N8 | 0,993208829 | 0,9524618 | 1,186757216 | 1,456706282 |
| N9 | 1,074702886 | 0,947368421 | 1,186757216 | 1,584040747 |
| **Average** | **1** | **0,971763613** | **1,196345148** | **1,445741431** |
| *SEM* | *0,01733543* | *0,011559488* | *0,01583802* | *0,043422454* |

### Conclusion:

The resistance was significantly increased in a dose dependent manner when endothelial cell monolayers were treated with NX218 at 10 and 100 µM for 1, 2 or 3 days compared with vehicle-treated endothelial cell monolayer.

### EXAMPLE 4: NX218 effect on the permeability of mouse brain endothelial cell monolayer

To determine the effect of NX218 on the permeability of mouse brain endothelial cells to a 40-kDa FITC Dextran, cells were grown on a semi-permeable membrane inserted in culture wells in presence or absence of NX218. Then, the FITC Dextran was placed on the apical chamber (containing the cells) and its levels measured (based on its fluorescence) one hour later in the basolateral chamber.

### Cell culture:

Mouse brain endothelial cells (bEnd.3, American Type Culture Collection) were cultured in DMEM supplemented with 10% FBS and 2 mM sodium pyruvate in a 5% CO₂ incubator at 37°C.

### Permeability assay:

bEnd.3 cells were grown to confluence on 1% fibronectin-coated Corning HTS 24-well Transwell polyester inserts with a pore size of 0.4 µm (5 × 10⁴ cells per well containing 200 µL and 800 µL of culture medium in the apical and basolateral compartments respectively) and treated with vehicle or NX218 (1, 10 and 100 µM) for 1, 2 or 3 days. Two hundred (200) µL of 1 mg/mL of a 40-kDa FITC Dextran in DMEM supplemented with 10 % FBS and 2 mM sodium pyruvate were added to the apical chamber of each well, and the cells were incubated at 37°C. Sampling aliquots were taken from the basolateral chamber and replaced with fresh medium every 15 min for 1 h and then transferred to 96-well plates. FITC-Dextran fluorescence was determined using a spectrofluorometer at an excitation wavelength of 485 nm and an emission wavelength of 520 nm. Relative fluorescence units were converted to values of nanograms per milliliter, using FITC-Dextran standard curves, and was corrected for background fluorescence and serial dilutions over the course of the experiment. The data are expressed as the endothelial permeability coefficient (Pe) in cm/min.

### Results:

The results are presented in Figure 2 and in Tables 4, 5 and 6 below.

**Table 4: Permeability of mouse brain endothelial cell monolayer to FITC-40 kDa Dextran after exposure to vehicle or different doses of NX218 (1, 10, 100 µM) for 24 hours**

| | **Control** | **NX218 1 µM** | **NX218 10 µM** | **NX218 100 µM** |
|---|---|---|---|---|
| N1 | 0,063 | 0,0612 | 0,05352 | 0,0544044 |
| N2 | 0,0696 | 0,05826 | 0,0618 | 0,060732 |
| N3 | 0,0654 | 0,072 | 0,0606 | 0,05437746 |
| **Average** | **0,066** | **0,06382** | **0,05864** | **0,05650462** |
| *SEM* | *0,00192873* | *0,004177128* | *0,002583331* | *0,002113704* |

**Table 5: Permeability of mouse brain endothelial cell monolayer to FITC-40 kDa Dextran after exposure to vehicle or different doses of NX218 (1, 10, 100 uM) for 48 hours**

| | **Control** | **NX218 1 µM** | **NX218 10 µM** | **NX218 100 µM** |
|---|---|---|---|---|
| N1 | 0,05988 | 0,05778228 | 0,05454 | 0,03762 |
| N2 | 0,05706 | 0,04689204 | 0,0456 | 0,0435 |
| N3 | 0,05154 | 0,05665896 | 0,0513 | 0,04518 |
| **Average** | **0,05616** | **0,05377776** | **0,05048** | **0,0421** |
| *SEM* | *0,00244924* | *0,0034581* | *0,002613121* | *0,002291899* |

**Table 6: Permeability of mouse brain endothelial cell monolayer to FITC-40 kDa Dextran after exposure to vehicle or different doses of NX218 (1, 10, 100 uM) for 72 hours**

| | **Control** | **NX218 1 µM** | **NX218 10 µM** | **NX218 100 µM** |
|---|---|---|---|---|
| N1 | 0,05062404 | 0,05651976 | 0,04488678 | 0,04790352 |
| N2 | 0,05442732 | 0,05653134 | 0,04697766 | 0,04552458 |
| N3 | 0,05474874 | 0,05009172 | 0,04271514 | 0,04726332 |
| **Average** | **0,0532667** | **0,05438094** | **0,04485986** | **0,04689714** |
| *SEM* | *0,00132458* | *0,002144613* | *0,001230557* | *0,000710728* |

### Conclusion:

A strong reduction of the permeability to the FITC dextran was observed for endothelial cell monolayers treated with NX218 in a dose-dependent manner, in particular for the highest doses after 2 or 3 days of treatment.

### EXAMPLE 5: NX218 effect on the expression of Claudin-5 at tight junction in mouse brain endothelial cell cultures

To understand the effect of NX218 on the TEER and permeability of mouse brain endothelial cells, the levels of the tight junction protein Claudin-5 were assessed using immunocytochemistry.

### Cell culture:

Mouse brain endothelial cells (bEnd.3, American Type Culture Collection) were cultured in DMEM supplemented with 10% fetal FBS and 2 mM sodium pyruvate in a 5% CO₂ incubator at 37°C.

### Immunocytochemistry:

bEnd.3 cells were seeded on 1% fibronectin-coated Nunc Lab-Tek II Chamber Slides in culture medium (1 × 10⁵ cells per well in 300 µL). Two days after seeding, cells were treated with vehicle or NX218 (1, 10 and 100 µM) for 1, 2 or 3 days. Cells were then fixed for 10 min at room temperature (RT) with 4% paraformaldehyde in phosphate buffered saline (PBS), washed twice with PBS and incubated with 5% normal goat serum and 0.05% Triton X-100 in PBS for 1 h at RT before overnight incubation with polyclonal rabbit anti-claudin-5 (1/100; 34-1600, Invitrogen) at 4°C. Cells were then washed three times with PBS and incubated with goat anti-rabbit Cy3 secondary antibody (1/500; ab6939, Abcam) for 2 h at RT and counterstained with Hoechst 33258 to visualize nuclei. The slide was removed from the chamber and mounted with Aqua Poly/Mount before visualization by confocal laser scanning /microscopy. Claudin-5 immunoreactivity (intensity) at membrane localization was finally quantified to evaluate Claudin-5 protein levels. The data are expressed as a percentage of the control condition.

### Results:

The results are presented in Figure 3 and in Tables 7, 8 and 9 below.

**Table 7: Expression of Claudin-5 at tight junction of mouse brain endothelial cell monolayer after exposure to vehicle or different doses of NX218 (1, 10, 100 uM) for 24 hours (% of Control)**

| | **Control** | **NX218 1 µM** | **NX218 10 µM** | **NX218 100 µM** |
|---|---|---|---|---|
| N1 | 72,440383 | 99,6064103 | 121,255365 | 129,044153 |
| N2 | 99,4293576 | 140,445096 | 169,426532 | 161,74853 |
| N3 | 122,5602 | 107,549505 | 200,894163 | 157,132374 |
| N4 | 82,1087374 | 108,061079 | 126,876682 | 173,250356 |
| N5 | 105,199357 | 99,9574488 | 154,448949 | 212,520386 |
| N6 | 118,261964 | 119,313375 | 137,025706 | 249,467751 |
| **Average** | **100** | **112,488819** | **151,6545662** | **180,5272583** |
| *SEM* | *8,06112206* | *6,311685949* | *12,24778588* | *17,68217223* |

**Table 8: Expression of Claudin-5 at tight junction of mouse brain endothelial cell monolayer after exposure to vehicle or different doses of NX218 (1, 10, 100 uM) for 48 hours (% of Control)**

| | **Control** | **NX218 1 µM** | **NX218 10 µM** | **NX218 100 µM** |
|---|---|---|---|---|
| N1 | 26,2625911 | 93,8815295 | 145,684038 | 122,734778 |
| N2 | 138,295957 | 102,090107 | 97,5141516 | 115,482876 |
| N3 | 145,17811 | 82,8922133 | 73,2520043 | 130,058504 |
| N4 | 93,1624188 | 76,0661471 | 66,3567517 | 88,3914396 |
| N5 | 92,5247649 | 52,4546378 | 83,17784 | 55,1787689 |
| N6 | 104,576159 | 76,3689044 | 120,828116 | 80,8659316 |
| **Average** | **100** | **80,62558985** | **97,80215027** | **98,78538302** |
| *SEM* | *17,3862209* | *7,00973952* | *12,42087815* | *11,77718908* |

**Table 9: Expression of Claudin-5 at tight junction of mouse brain endothelial cell monolayer after exposure to vehicle or different doses of NX218 (1, 10, 100 uM) for 72 hours (% of Control)**

| | **Control** | **NX218 1 µM** | **NX218 10 µM** | **NX218 100 µM** |
|---|---|---|---|---|
| N1 | 87,225305 | 90,2294853 | 94,6288633 | 116,000124 |
| N2 | 98,8335973 | 103,833343 | 58,4195654 | 81,0324611 |
| N3 | 101,793386 | 55,5329838 | 71,8566143 | 107,669132 |
| N4 | 112,147711 | 77,152673 | 56,0273654 | 121,824456 |
| **Average** | **100** | **81,68712128** | **70,2331021** | **106,6315433** |
| *SEM* | *5,12621078* | *10,27953974* | *8,846616872* | *9,013806542* |

### Conclusion:

Although the levels were similar between control and NX218 conditions after 2 or 3 days treatment, a significant increase in Claudin-5 levels was observed when endothelial cell monolayers were treated for 24 hours with NX218 at 10 and 100 µM. It is therefore likely that the increased level of Claudin-5 induced by NX218 contribute to the increase in TEER and to the decrease in permeability observed of mouse endothelial brain cells in presence of NX218.

### EXAMPLE 6: NX218 effect on the permeability of primary rat choroid plexus epithelial cell monolayer

To determine the effect of NX218 on the permeability of primary rat choroid plexus epithelial cell monolayer to radiolabeled sucrose, cells were grown on a semi-permeable membrane inserted in culture wells in presence or absence of NX218. [14C]-sucrose was then placed on the apical chamber (containing the cells) and it was quantified up to 2 hours later in the basolateral chamber.

Choroid plexus epithelial cells (CPECs) were prepared from lateral ventricle choroid plexus isolated from rat and cultured as described previously (Strazielle et al., 1999). Cells were plated on polycarbonate membranes (0.4 µM pore size, 0.33 cm² growth area), and then exposed on their blood-facing membrane to NX218 (0, 250, and 1000 µg/mL) and labeled sucrose for 2 hours at 37°C. Aliquots from the acceptor compartment were sampled repeatedly (after 12, 25, 40, 60, 90 and 120 min).

### Results:

The results are presented in Figure 4 and in Tables 10 and 11 below.

**Table 10: Permeability of rat choroid plexus epithelial cell monolayer to labeled sucrose after exposure to NX218 (192 and 768 µM) 0-90 minutes period**

| | **Control** | **NX218 192 µM** | **NX218 768 µM** |
|---|---|---|---|
| N1 | 0,0936 | 0,0858 | 0,0768 |
| N2 | 0,0973 | 0,0889 | 0,0787 |
| N3 | 0,092 | 0,0899 | 0,0759 |
| N4 | 0,0883 | 0,0911 | 0,0759 |
| **Average** | **0,0928** | **0,088925** | **0,076825** |
| *SEM* | *0,001865922* | *0,001134589* | *0,000660019* |

**Table 11: Permeability of rat choroid plexus epithelial cell monolayer to labeled sucrose after exposure to NX218 (192 and 768 µM) 90-120 minutes period**

| | **Control** | **NX218 192 µM** | **NX218 768 µM** |
|---|---|---|---|
| N1 | 0,4019 | 0,2571 | 0,1239 |
| N2 | 0,4262 | 0,2283 | 0,127 |
| N3 | 0,2988 | 0,3182 | 0,1082 |
| N4 | 0,3844 | 0,3583 | 0,1257 |

| **Average** | **0,377825** | **0,290475** | **0,1212** |
|---|---|---|---|
| *SEM* | *0,027700673* | *0,029366261* | *0,004379688* |

### Conclusion:

A strong reduction of the permeability to the [14C]-sucrose was observed for epithelial cell monolayers treated with NX218 at the highest dose (768 µM) as early as after 1h30 of treatment.

### References (incorporated herein bv reference):

Abdullahi, W., Tripathi, D., & Ronaldson, P. T. (2018). Blood-brain barrier dysfunction in ischemic stroke: targeting tight junctions and transporters for vascular protection. Am J Physiol Cell Physiol, 315(3), C343-C356. doi: 10.1152/ajpcell.00095.2018
Arima, M., Nakao, S., Yamaguchi, M., Feng, H., Fujii, Y., Shibata, K., et al. (2020). Claudin-5 Redistribution Induced by Inflammation Leads to Anti-VEGF-Resistant Diabetic Macular Edema. Diabetes, 69(5), 981-999. doi: 10.2337/db19-1121
Bartanusz, V., Jezova, D., Alajajian, B., & Digicaylioglu, M. (2011). The blood-spinal cord barrier: morphology and clinical implications. Ann Neurol, 70(2), 194-206. doi: 10.1002/ana.22421
Berghoff, S. A., Düking, T., Spieth, L., Winchenbach, J., Stumpf, S. K., Gerndt, N., et al. (2017). Blood-brain barrier hyperpermeability precedes demyelination in the cuprizone model. Acta Neuropathol Commun, 5(1), 94. doi: 10.1186/s40478-017-0497-6
Buzhdygan, T. P., DeOre, B. J., Baldwin-Leclair, A., Bullock, T. A., McGary, H. M., Khan, J. A., et al. (2020). The SARS-CoV-2 spike protein alters barrier function in 2D static and 3D microfluidic in-vitro models of the human blood-brain barrier. Neurobiol Dis, 146, 105131. doi: 10.1016/j.nbd.2020.105131
Cash, A., & Theus, M. H. (2020). Mechanisms of Blood-Brain Barrier Dysfunction in Traumatic Brain Injury. Int J Mol Sci, 21(9). doi: 10.3390/ijms21093344
Chen, W., Sharma, R., Rizzo, A. N., Siegler, J. H., Garcia, J. G., & Jacobson, J. R. (2014). Role of claudin-5 in the attenuation of murine acute lung injury by simvastatin. Am J Respir Cell Mol Biol, 50(2), 328-336. doi: 10.1165/rcmb.2013-0058OC
Evran, S., Calis, F., Akkaya, E., Baran, O., Cevik, S., Katar, S., et al. (2020). The effect of high mobility group box-1 protein on cerebral edema, blood-brain barrier, oxidative stress and apoptosis in an experimental traumatic brain injury model. Brain Res Bull, 154, 68-80. doi: 10.1016/j.brainresbull.2019.10.013
Greene, C., Hanley, N., & Campbell, M. (2019). Claudin-5: gatekeeper of neurological function. Fluids Barriers CNS, 16(1), 3. doi: 10.1186/s12987-019-0123-z
Greene, C., Kealy, J., Humphries, M. M., Gong, Y., Hou, J., Hudson, N., et al. (2018). Dose-dependent expression of claudin-5 is a modifying factor in schizophrenia. Mol Psychiatry, 23(11), 2156-2166. doi: 10.1038/mp.2017.156
Hashimoto, Y., Campbell, M., Tachibana, K., Okada, Y., & Kondoh, M. (2021). Claudin-5: A Pharmacological Target to Modify the Permeability of the Blood-Brain Barrier. Biol Pharm Bull, 44(10), 1380-1390. doi: 10.1248/bpb.b21-00408
Huang, L. Y., Stuart, C., Takeda, K., D'Agnillo, F., & Golding, B. (2016). Poly(I:C) Induces Human Lung Endothelial Barrier Dysfunction by Disrupting Tight Junction Expression of Claudin-5. PLoS One, 11(8), e0160875. doi: 10.1371/journal.pone.0160875
Hudson, N., Celkova, L., Hopkins, A., Greene, C., Storti, F., Ozaki, E., et al. (2019). Dysregulated claudin-5 cycling in the inner retina causes retinal pigment epithelial cell atrophy. JCI Insight, 4(15). doi: 10.1172/jci.insight.130273
Kakaroubas, N., Brennan, S., Keon, M., & Saksena, N. K. (2019). Pathomechanisms of Blood-Brain Barrier Disruption in ALS. Neurosci J, 2019, 2537698. doi: 10.1155/2019/2537698
Liddelow, S. A. (2015). Development of the choroid plexus and blood-CSF barrier. Front Neurosci, 9, 32. doi: 10.3389/fnins.2015.00032
Lv, J., Hu, W., Yang, Z., Li, T., Jiang, S., Ma, Z., et al. (2018). Focusing on claudin-5: A promising candidate in the regulation of BBB to treat ischemic stroke. Prog Neurobiol, 161, 79-96. doi: 10.1016/j.pneurobio.2017.12.001
Meister, S., Storck, S. E., Hameister, E., Behl, C., Weggen, S., Clement, A. M., & Pietrzik, C. U. (2015). Expression of the ALS-causing variant hSOD1 (G93A) leads to an impaired integrity and altered regulation of claudin-5 expression in an in vitro blood-spinal cord barrier model. J Cereb Blood Flow Metab, 35(7), 1112-1121. doi: 10.1038/jcbfm.2015.57
Montagne, A., Nation, D. A., Sagare, A. P., Barisano, G., Sweeney, M. D., Chakhoyan, A., et al. (2020). APOE4 leads to blood-brain barrier dysfunction predicting cognitive decline. Nature, 581(7806), 71-76. doi: 10.1038/s41586-020-2247-3
Pellegrini, L., Albecka, A., Mallery, D. L., Kellner, M. J., Paul, D., Carter, A. P., et al. (2020). SARS-CoV-2 Infects the Brain Choroid Plexus and Disrupts the Blood-CSF Barrier in Human Brain Organoids. Cell Stem Cell, 27(6), 951-961.e955. doi: 10.1016/j.stem.2020.10.001
Profaci, C. P., Munji, R. N., Pulido, R. S., & Daneman, R. (2020). The blood-brain barrier in health and disease: Important unanswered questions. J Exp Med, 217(4). doi: 10.1084/jem.20190062
Solar, P., Zamani, A., Kubí 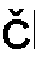 ková, L., Dubový, P., & Joukal, M. (2020). Choroid plexus and the blood-cerebrospinal fluid barrier in disease. Fluids Barriers CNS, 17(1), 35. doi: 10.1186/s12987-020-00196-2
Strazielle, N., & Ghersi-Egea, J. F. (1999). Demonstration of a coupled metabolism-efflux process at the choroid plexus as a mechanism of brain protection toward xenobiotics. J Neurosci, 19(15), 6275-6289.
Sweeney, M. D., Sagare, A. P., & Zlokovic, B. V. (2018). Blood-brain barrier breakdown in Alzheimer disease and other neurodegenerative disorders. Nat Rev Neurol, 14(3), 133-150. doi: 10.1038/nrneurol.2017.188
Sweeney, M. D., Zhao, Z., Montagne, A., Nelson, A. R., & Zlokovic, B. V. (2019). Blood-Brain Barrier: From Physiology to Disease and Back. Physiol Rev, 99(1), 21-78. doi: 10.1152/physrev.00050.2017
Uher, T., Horakova, D., Tyblova, M., Zeman, D., Krasulova, E., Mrazova, K., et al. (2016). Increased albumin quotient (QAlb) in patients after first clinical event suggestive of multiple sclerosis is associated with development of brain atrophy and greater disability 48 months later. Mult Scler, 22(6), 770-781. doi: 10.1177/1352458515601903
van der Wijk, A. E., Canning, P., van Heijningen, R. P., Vogels, I. M. C., van Noorden, C. J. F., Klaassen, I., & Schlingemann, R. O. (2019). Glucocorticoids exert differential effects on the endothelium in an in vitro model of the blood-retinal barrier. Acta Ophthalmol, 97(2), 214-224. doi: 10.1111/aos.13909
van Vliet, E. A., Ndode-Ekane, X. E., Lehto, L. J., Gorter, J. A., Andrade, P., Aronica, E., et al. (2020). Long-lasting blood-brain barrier dysfunction and neuroinflammation after traumatic brain injury. Neurobiol Dis, 145, 105080. doi: 10.1016/j.nbd.2020.105080
Yamazaki, Y., Shinohara, M., Yamazaki, A., Murray, M. E., Liesinger, A. M., Heckman, M. G., et al. (2019). Selective loss of cortical endothelial tight junction proteins during Alzheimer's disease progression. Brain, 142(4), 1077-1092. doi: 10.1093/brain/awz011

## Claims

1. A peptide of amino acid sequence
X1-W-S-A1-W-S-A2-C-S-A3-A4-C-G-X2 (SEQ ID NO: 1) in which :
- A1, A2, A3 and A4 consists of amino acid sequences consisting of 1 to 5 amino acids,
- X1 and X2 consists of amino acid sequences consisting of 1 to 6 amino acids; or X1 and X2 are absent;
- it being possible for the N-terminal amino acid to be acetylated, for the C-terminal amino acid to be amidated, or the N-terminal amino acid to be acetylated and the C-terminal amino acid to be amidated,
for use in treating a biological barrier dysfunction.

2. The peptide for the use of claim 1, wherein the biological barrier dysfunction comprises altered tight junctions between adjacent cells forming the endothelial or epithelial layers of a biological barrier; transendothelial and/or transepithelial electrical resistance decrease; deregulation of Claudin-5 expression or distribution; and/or the trans-compartment permeability deregulation of specific compounds or molecules.

3. The peptide for the use of claim 1 or 2, wherein the biological barrier is the blood-brain barrier, the blood-spinal cord barrier, the inner blood-retina barrier, the blood-cerebrospinal fluid barrier, the lung endothelial barrier, a vascular-parenchyma or a vascular-fluid barrier.

4. The peptide for the use of any one of the preceding claims, wherein the biological barrier dysfunction is one occurring in disease or conditions comprising vascular diseases, infections, immune system hyper-responsiveness, inflammatory diseases or autoimmune diseases, chronic kidney disease, intestinal diseases, macular degeneration, diabetic macular edema, epilepsy, CNS diseases and disorders, and aging.

5. The peptide for the use of any one of the preceding claims, wherein the peptide is of amino acid sequence W-S-A1-W-S-A2-C-S-A3-A4-C-G (SEQ ID NO: 2), in which A1, A2, A3 and A4 consists of amino acid sequences consisting of 1 to 5 amino acids.

6. The peptide for the use of any one of the preceding claims, wherein
- A1 is chosen from G, V, S, P and A, preferably G, S,
- A2 is chosen from G, V, S, P and A, preferably G, S,
- A3 is chosen from R, A and V, preferably R, V, and/or
- A4 is chosen from S, T, P and A, preferably S, T.

7. The peptide for the use of any one of the preceding claims, wherein A1 and A2 are independently chosen from G and S, and/or A3-A4 is chosen from R-S or V-S or V-T or R-T.

8. The peptide for the use of any one of the preceding claims, wherein the peptide is of a sequence selected from the group consisting of sequences SEQ ID NO: 3- 63.

9. The peptide for the use of any one of the preceding claims, wherein the peptide is a linear peptide or a cyclized peptide wherein the two cysteines appearing on the peptide formula of SEQ ID NO: 1 or 2 form a disulfide bridge.

10. The peptide for the use of any one of claims 1 to 4, which is the peptide of sequence SEQ ID NO: 3, wherein the peptide is a linear peptide or a cyclized peptide wherein the two cysteines form a disulfide bridge, or a mixture of both.

11. The peptide for the use of any one of claims 1 to 4, which is the peptide of sequence SEQ ID NO: 3, wherein the peptide is a cyclized peptide wherein the two cysteines form a disulfide bridge.

12. The peptide for the use of any one of the preceding claims, wherein the peptide is for use in a patient who has been determined as having a biological barrier dysfunction, deteriorated tight junctions, abnormal Claudin-5 distribution, deregulated transendothelial and/or transepithelial electrical resistance at the level of the biological barrier, the trans-compartment permeability deregulation of specific compounds or molecules.


SEQUENCE LISTING

<110>  AXOLTIS PHARMA

<120>  SCO-spondin-derived polypeptides for treating biological barriers
dysfunction

<130>  BET 21L2845

<160>  63

<170>  PatentIn version 3.5

<210>  1
<211>  14
<212>  PRT
<213>  artificial sequence

<220>
<223>  Peptide compound



<220>
<221>  MISC_FEATURE
<222>  (1)..(1)
<223>  Xaa  is absent or 1 to 6 amino acids

<220>
<221>  MISC_FEATURE
<222>  (4)..(4)
<223>  Xaa  is 1 to 5 amino acids

<220>
<221>  MISC_FEATURE
<222>  (7)..(7)
<223>  Xaa is 1 to 5 amino acids

<220>
<221>  MISC_FEATURE
<222>  (10)..(11)
<223>  Xaa is 1 to 5 amino acids

<220>
<221>  MISC_FEATURE
<222>  (14)..(14)
<223>  Xaa is  absent or is 1 to 6 amino acids

<400>  1

Xaa Trp Ser Xaa Trp Ser Xaa Cys Ser Xaa Xaa Cys Gly Xaa
1               5                   10


<210>  2
<211>  12
<212>  PRT
<213>  Artificial sequence

<220>
<223>  Peptide compound


<220>

<221>  MISC_FEATURE
<222>  (3)..(3)
<223>  Xaa is 1 to 5 amino acids

<220>
<221>  MISC_FEATURE
<222>  (6)..(6)
<223>  Xaa is 1 to 5 amino acids

<220>
<221>  MISC_FEATURE
<222>  (9)..(10)
<223>  Xaa is 1 to 5 amino acids

<400>  2

Trp Ser Xaa Trp Ser Xaa Cys Ser Xaa Xaa Cys Gly
1               5                   10


<210>  3
<211>  12
<212>  PRT
<213>  Artificial sequence

<220>
<223>  Peptide compound

<400>  3

Trp Ser Gly Trp Ser Ser Cys Ser Arg Ser Cys Gly
1               5                   10


<210>  4
<211>  12
<212>  PRT
<213>  Artificial sequence

<220>
<223>  Peptide compound

<400>  4

Trp Ser Ser Trp Ser Gly Cys Ser Arg Ser Cys Gly
1               5                   10


<210>  5
<211>  12
<212>  PRT
<213>  artificial sequence

<220>
<223>  peptide compound

<400>  5

Trp Ser Ser Trp Gly Ser Cys Ser Arg Ser Cys Gly
1               5                   10


<210>  6

<211>  12
<212>  PRT
<213>  artificial sequence

<220>
<223>  peptide compound

<400>  6

Trp Ser Ser Trp Gly Gly Cys Ser Arg Ser Cys Gly
1               5                   10


<210>  7
<211>  12
<212>  PRT
<213>  artificial sequence

<220>
<223>  peptide compound

<400>  7

Trp Ser Ser Trp Ser Ser Cys Ser Arg Ser Cys Gly
1               5                   10


<210>  8
<211>  12
<212>  PRT
<213>  artificial sequence

<220>
<223>  peptide compound

<400>  8

Trp Ser Gly Trp Ser Gly Cys Ser Arg Ser Cys Gly
1               5                   10


<210>  9
<211>  12
<212>  PRT
<213>  artificial sequence

<220>
<223>  peptide compound

<400>  9

Trp Ser Gly Trp Gly Ser Cys Ser Arg Ser Cys Gly
1               5                   10


<210>  10
<211>  12
<212>  PRT
<213>  artificial sequence

<220>
<223>  peptide compound

<400>  10

Trp Ser Gly Trp Gly Gly Cys Ser Arg Ser Cys Gly
1               5                   10


<210>  11
<211>  12
<212>  PRT
<213>  artificial sequence

<220>
<223>  peptide compound

<400>  11

Trp Ser Ser Trp Ser Ser Cys Ser Val Ser Cys Gly
1               5                   10


<210>  12
<211>  12
<212>  PRT
<213>  artificial sequence

<220>
<223>  peptide compound

<400>  12

Trp Ser Ser Trp Ser Gly Cys Ser Val Ser Cys Gly
1               5                   10


<210>  13
<211>  12
<212>  PRT
<213>  artificial sequence

<220>
<223>  peptide compound

<400>  13

Trp Ser Ser Trp Gly Ser Cys Ser Val Ser Cys Gly
1               5                   10


<210>  14
<211>  12
<212>  PRT
<213>  artificial sequence

<220>
<223>  peptide compound

<400>  14

Trp Ser Ser Trp Gly Gly Cys Ser Val Ser Cys Gly
1               5                   10


<210>  15

<211>  12
<212>  PRT
<213>  artificial sequence

<220>
<223>  peptide compound

<400>  15

Trp Ser Gly Trp Ser Ser Cys Ser Val Ser Cys Gly
1               5                   10


<210>  16
<211>  12
<212>  PRT
<213>  artificial sequence

<220>
<223>  peptide compound

<400>  16

Trp Ser Gly Trp Ser Gly Cys Ser Val Ser Cys Gly
1               5                   10


<210>  17
<211>  12
<212>  PRT
<213>  artificial sequence

<220>
<223>  peptide compound

<400>  17

Trp Ser Gly Trp Gly Ser Cys Ser Val Ser Cys Gly
1               5                   10


<210>  18
<211>  12
<212>  PRT
<213>  artificial sequence

<220>
<223>  peptide compound

<400>  18

Trp Ser Gly Trp Gly Gly Cys Ser Val Ser Cys Gly
1               5                   10


<210>  19
<211>  12
<212>  PRT
<213>  artificial sequence

<220>
<223>  peptide compound

<400>  19

Trp Ser Ser Trp Ser Ser Cys Ser Val Thr Cys Gly
1               5                   10


<210>  20
<211>  12
<212>  PRT
<213>  artificial sequence

<220>
<223>  peptide compound

<400>  20

Trp Ser Ser Trp Ser Gly Cys Ser Val Thr Cys Gly
1               5                   10


<210>  21
<211>  12
<212>  PRT
<213>  artificial sequence

<220>
<223>  peptide compound

<400>  21

Trp Ser Ser Trp Gly Ser Cys Ser Val Thr Cys Gly
1               5                   10


<210>  22
<211>  12
<212>  PRT
<213>  artificial sequence

<220>
<223>  peptide compound

<400>  22

Trp Ser Ser Trp Gly Gly Cys Ser Val Thr Cys Gly
1               5                   10


<210>  23
<211>  12
<212>  PRT
<213>  artificial sequence

<220>
<223>  peptide compound

<400>  23

Trp Ser Gly Trp Ser Ser Cys Ser Val Thr Cys Gly
1               5                   10


<210>  24

<211>  12
<212>  PRT
<213>  Artificial Sequence

<220>
<223>  peptide compound

<400>  24

Trp Ser Gly Trp Ser Gly Cys Ser Val Thr Cys Gly
1               5                   10


<210>  25
<211>  12
<212>  PRT
<213>  Artificial Sequence

<220>
<223>  peptide compound

<400>  25

Trp Ser Gly Trp Gly Ser Cys Ser Val Thr Cys Gly
1               5                   10


<210>  26
<211>  12
<212>  PRT
<213>  Artificial Sequence

<220>
<223>  peptide compound

<400>  26

Trp Ser Gly Trp Gly Gly Cys Ser Val Thr Cys Gly
1               5                   10


<210>  27
<211>  12
<212>  PRT
<213>  Artificial Sequence

<220>
<223>  peptide compound

<400>  27

Trp Ser Ser Trp Ser Ser Cys Ser Arg Thr Cys Gly
1               5                   10


<210>  28
<211>  12
<212>  PRT
<213>  artificial sequence

<220>
<223>  peptide compound

<400>  28

Trp Ser Ser Trp Ser Gly Cys Ser Arg Thr Cys Gly
1               5                   10


<210>  29
<211>  12
<212>  PRT
<213>  Artificial Sequence

<220>
<223>  peptide compound

<400>  29

Trp Ser Ser Trp Gly Ser Cys Ser Arg Thr Cys Gly
1               5                   10


<210>  30
<211>  12
<212>  PRT
<213>  Artificial Sequence

<220>
<223>  peptide compound

<400>  30

Trp Ser Ser Trp Gly Gly Cys Ser Arg Thr Cys Gly
1               5                   10


<210>  31
<211>  12
<212>  PRT
<213>  Artificial Sequence

<220>
<223>  peptide compound

<400>  31

Trp Ser Gly Trp Ser Ser Cys Ser Arg Thr Cys Gly
1               5                   10


<210>  32
<211>  12
<212>  PRT
<213>  Artificial Sequence

<220>
<223>  peptide compound

<400>  32

Trp Ser Gly Trp Ser Gly Cys Ser Arg Thr Cys Gly
1               5                   10


<210>  33

<211>  12
<212>  PRT
<213>  Artificial Sequence

<220>
<223>  peptide compound

<400>  33

Trp Ser Gly Trp Gly Ser Cys Ser Arg Thr Cys Gly
1               5                   10


<210>  34
<211>  12
<212>  PRT
<213>  Artificial Sequence

<220>
<223>  peptide compound

<400>  34

Trp Ser Gly Trp Gly Gly Cys Ser Arg Thr Cys Gly
1               5                   10


<210>  35
<211>  13
<212>  PRT
<213>  Artificial Sequence

<220>
<223>  peptide compound

<400>  35

Pro Trp Ser Gly Trp Ser Ser Cys Ser Arg Ser Cys Gly
1               5                   10


<210>  36
<211>  14
<212>  PRT
<213>  Artificial Sequence

<220>
<223>  peptide compound

<400>  36

Ala Pro Trp Ser Gly Trp Ser Ser Cys Ser Arg Ser Cys Gly
1               5                   10


<210>  37
<211>  15
<212>  PRT
<213>  Artificial Sequence

<220>
<223>  peptide compound

<400>  37

Leu Ala Pro Trp Ser Gly Trp Ser Ser Cys Ser Arg Ser Cys Gly
1               5                   10                  15


<210>  38
<211>  16
<212>  PRT
<213>  Artificial Sequence

<220>
<223>  peptide compound

<400>  38

Val Leu Ala Pro Trp Ser Gly Trp Ser Ser Cys Ser Arg Ser Cys Gly
1               5                   10                  15


<210>  39
<211>  13
<212>  PRT
<213>  Artificial Sequence

<220>
<223>  peptide compound

<400>  39

Trp Ser Gly Trp Ser Ser Cys Ser Arg Ser Cys Gly Leu
1               5                   10


<210>  40
<211>  14
<212>  PRT
<213>  Artificial Sequence

<220>
<223>  peptide compound

<400>  40

Trp Ser Gly Trp Ser Ser Cys Ser Arg Ser Cys Gly Leu Gly
1               5                   10


<210>  41
<211>  15
<212>  PRT
<213>  Artificial Sequence

<220>
<223>  peptide compound

<400>  41

Trp Ser Gly Trp Ser Ser Cys Ser Arg Ser Cys Gly Leu Gly Leu
1               5                   10                  15


<210>  42

<211>  16
<212>  PRT
<213>  Artificial Sequence

<220>
<223>  peptide compound

<400>  42

Trp Ser Gly Trp Ser Ser Cys Ser Arg Ser Cys Gly Leu Gly Leu Ile
1               5                   10                  15


<210>  43
<211>  17
<212>  PRT
<213>  Artificial Sequence

<220>
<223>  peptide compound

<400>  43

Trp Ser Gly Trp Ser Ser Cys Ser Arg Ser Cys Gly Leu Gly Leu Ile
1               5                   10                  15


Phe




<210>  44
<211>  14
<212>  PRT
<213>  Artificial Sequence

<220>
<223>  peptide compound

<400>  44

Pro Trp Ser Gly Trp Ser Ser Cys Ser Arg Ser Cys Gly Leu
1               5                   10


<210>  45
<211>  15
<212>  PRT
<213>  Artificial Sequence

<220>
<223>  peptide compound

<400>  45

Pro Trp Ser Gly Trp Ser Ser Cys Ser Arg Ser Cys Gly Leu Gly
1               5                   10                  15


<210>  46
<211>  16
<212>  PRT
<213>  Artificial Sequence

<220>
<223>  peptide compound

<400>  46

Pro Trp Ser Gly Trp Ser Ser Cys Ser Arg Ser Cys Gly Leu Gly Leu
1               5                   10                  15


<210>  47
<211>  17
<212>  PRT
<213>  Artificial Sequence

<220>
<223>  peptide compound

<400>  47

Pro Trp Ser Gly Trp Ser Ser Cys Ser Arg Ser Cys Gly Leu Gly Leu
1               5                   10                  15


Ile


<210>  48
<211>  18
<212>  PRT
<213>  Artificial Sequence

<220>
<223>  peptide compound

<400>  48

Pro Trp Ser Gly Trp Ser Ser Cys Ser Arg Ser Cys Gly Leu Gly Leu
1               5                   10                  15


Ile Phe


<210>  49
<211>  15
<212>  PRT
<213>  Artificial Sequence

<220>
<223>  peptide compound

<400>  49

Ala Pro Trp Ser Gly Trp Ser Ser Cys Ser Arg Ser Cys Gly Leu
1               5                   10                  15


<210>  50
<211>  16
<212>  PRT

<213>  Artificial Sequence

<220>
<223>  peptide compound

<400>  50

Ala Pro Trp Ser Gly Trp Ser Ser Cys Ser Arg Ser Cys Gly Leu Gly
1               5                   10                  15


<210>  51
<211>  17
<212>  PRT
<213>  Artificial Sequence

<220>
<223>  peptide compound

<400>  51

Ala Pro Trp Ser Gly Trp Ser Ser Cys Ser Arg Ser Cys Gly Leu Gly
1               5                   10                  15


Leu




<210>  52
<211>  18
<212>  PRT
<213>  Artificial Sequence

<220>
<223>  peptide compound

<400>  52

Ala Pro Trp Ser Gly Trp Ser Ser Cys Ser Arg Ser Cys Gly Leu Gly
1               5                   10                  15


Leu Ile




<210>  53
<211>  19
<212>  PRT
<213>  Artificial Sequence

<220>
<223>  peptide compound

<400>  53

Ala Pro Trp Ser Gly Trp Ser Ser Cys Ser Arg Ser Cys Gly Leu Gly
1               5                   10                  15


Leu Ile Phe

<210>  54
<211>  16
<212>  PRT
<213>  Artificial Sequence

<220>
<223>  peptide compound

<400>  54

Leu Ala Pro Trp Ser Gly Trp Ser Ser Cys Ser Arg Ser Cys Gly Leu
1               5                   10                  15


<210>  55
<211>  17
<212>  PRT
<213>  Artificial Sequence

<220>
<223>  peptide compound

<400>  55

Leu Ala Pro Trp Ser Gly Trp Ser Ser Cys Ser Arg Ser Cys Gly Leu
1               5                   10                  15


Gly




<210>  56
<211>  18
<212>  PRT
<213>  Artificial Sequence

<220>
<223>  peptide compound

<400>  56

Leu Ala Pro Trp Ser Gly Trp Ser Ser Cys Ser Arg Ser Cys Gly Leu
1               5                   10                  15


Gly Leu




<210>  57
<211>  19
<212>  PRT
<213>  Artificial Sequence

<220>
<223>  peptide compound

<400>  57

Leu Ala Pro Trp Ser Gly Trp Ser Ser Cys Ser Arg Ser Cys Gly Leu

1               5                   10                  15

Gly Leu Ile

<210>  58
<211>  20
<212>  PRT
<213>  Artificial Sequence

<220>
<223>  peptide compound

<400>  58

Leu Ala Pro Trp Ser Gly Trp Ser Ser Cys Ser Arg Ser Cys Gly Leu
1               5                   10                  15

Gly Leu Ile Phe
            20

<210>  59
<211>  17
<212>  PRT
<213>  Artificial Sequence

<220>
<223>  peptide compound

<400>  59

Val Leu Ala Pro Trp Ser Gly Trp Ser Ser Cys Ser Arg Ser Cys Gly
1               5                   10                  15

Leu

<210>  60
<211>  18
<212>  PRT
<213>  Artificial Sequence

<220>
<223>  peptide compound

<400>  60

Val Leu Ala Pro Trp Ser Gly Trp Ser Ser Cys Ser Arg Ser Cys Gly
1               5                   10                  15

Leu Gly

<210>  61
<211>  19

<212>  PRT
<213>  Artificial Sequence

<220>
<223>  peptide compound

<400>  61

Val Leu Ala Pro Trp Ser Gly Trp Ser Ser Cys Ser Arg Ser Cys Gly
1               5                   10                  15

Leu Gly Leu

<210>  62
<211>  20
<212>  PRT
<213>  Artificial Sequence

<220>
<223>  peptide compound

<400>  62

Val Leu Ala Pro Trp Ser Gly Trp Ser Ser Cys Ser Arg Ser Cys Gly
1               5                   10                  15

Leu Gly Leu Ile
            20

<210>  63
<211>  21
<212>  PRT
<213>  Artificial Sequence

<220>
<223>  peptide compound

<400>  63

Val Leu Ala Pro Trp Ser Gly Trp Ser Ser Cys Ser Arg Ser Cys Gly
1               5                   10                  15

Leu Gly Leu Ile Phe
            20